# FASCICULE DE BREVET EUROPEEN

(11) **EP 0 972 518 B1**
(45) Date de publication et mention de la délivrance du brevet: **24.11.2004**
(21) Numéro de dépôt: 99401724.2
(22) Date de dépôt: 08.07.1999
(51) Int. Cl.: A61K 35/14, C12N 5/10, A61K 39/395

(54) **Echange de lymphocytes T**
Austausch von T-Lymphozyten
Exchange of T lymphocytes

(30) Priorité: 10.07.1998 FR 9808952
(43) Date de publication de la demande: 19.01.2000
(73) Titulaire: UNIVERSITE PIERRE ET MARIE CURIE PARIS VI, 75252 Paris Cédex 05 (FR)
(72) Inventeur: Klatzmann, David, 75013 Paris (FR); Cohen, José, 75020 Paris (FR); Boyer, Olivier, 75018 Paris (FR); Thomas-Vaslin, Véronique, 94320 Thiais (FR); Salzmann, Jean-Loup, 75005 Paris (FR)
(74) Mandataire: Becker, Philippe

(56) Documents cités:
- WO-A-96/05287
- WO-A-97/45142
- CONTASSOT E ET AL: "In vivo alloreactive potential of ex vivo-expanded primary T lymphocytes." TRANSPLANTATION, (1998 MAY 27) 65 (10) 1365-70, XP002100637
- TIBERGHIEN, PIERRE: "Use of donor T - lymphocytes expressing herpes-simplex thymidine kinase in allogeneic bone marrow transplantation: a phase I-II study" HUM. GENE THER. (1997), 8(5), 615-624, XP002100638
- COHEN J L ET AL: "Prevention of graft-versus-host disease in mice using a suicide gene expressed in T lymphocytes." BLOOD, (1997 JUN 15) 89 (12) 4636-45. , XP002123704
- GEORGES G E ET AL: "CANINE CTL TRANSDUCED WITH HS-THYMIDINE KINASE GENE MAINTAIN CYTOTOXIC ACTIVITY AND ARE ELIMINATED BY GANCICLOVIR: A MODEL TO ENHANCE ENGRAFTMENT, CONTROL GVHD AND DECREASE PREPARATIVE REGIMEN INTENSITY IN ALLOGENEIC BLOOD STEM CELL TRANSPLANTATION" BLOOD,US,PHILADELPHIA, PA, vol. 88, no. 10, SUPPL. 01, page 245A XP000670304 ISSN: 0006-4971
- TIBERGHIEN P: "[Modulation of alloreactivity using genetically modified T lymphocytes ]. Modulation de l'alloreactivite par l'utilisation de lymphocytes T genetiquement modifies." TRANSFUSION CLINIQUE ET BIOLOGIQUE, (1997) 4 (3) 275-80, XP002100639
- HELENE M ET AL: "Inhibition of graft-versus-host disease. Use of a T cell-controlled suicide gene." JOURNAL OF IMMUNOLOGY, (1997 JUN 1) 158 (11) 5079-82, XP002100640
- BORDIGNON C ET AL: "Transfer of the HSV-tk gene into donor peripheral blood lymphocytes for in vivo modulation of donor anti-tumor immunity after allogeneic bone marrow transplantation." HUMAN GENE THERAPY, (1995 JUN) 6 (6) 813-9, XP002100641
- GEORGES G E ET AL: "Canine T cells transduced with a herpes simplex virus thymidine kinase gene: a model to study effects on engraftment and control of graft-versus-host disease." TRANSPLANTATION, (1998 AUG 27) 66 (4) 540-4, XP000857093
- TIBERGHIEN P: ""Suicide" gene for the control of graft-versus-host disease." CURRENT OPINION IN HEMATOLOGY, (1998 NOV) 5 (6) 478-82, XP000857091

## Description

La présente invention concerne les domaines de l'immunologie et de la médecine. Elle concerne plus particulièrement une nouvelle approche immunologique permettant le traitement de nombreuses pathologies. L'invention repose plus particulièrement sur une procédure "d'échange de lymphocytes T" à des fins thérapeutiques, après déplétion des lymphocytes T de l'hôte. L'invention peut être appliquée au traitement de pathologies nombreuses, dans lesquelles les lymphocytes T sont impliqués, telles que les maladies autoimmunes, les greffes, les maladies virales, l'allergie, etc.

Les lymphocytes T sont des cellules essentielles pour développer les réponses immunitaires efficaces qui permettent de protéger l'organisme notamment contre les agents infectieux ou les cellules tumorales. Cependant, si la réponse des lymphocytes T est le plus souvent bénéfique, dans certaines situations elle peut au contraire être source de pathologies (maladies autoimmunes, rejets de greffes, etc.). Dans ces situations il est donc particulièrement important de pouvoir contrôler la réponse des lymphocytes T afin de traiter l'immunopathologie.

Il existe plusieurs thérapeutiques actuellement utilisées dans le but de contrôler les réponses immunitaires T lorsque cela est nécessaire. Le traitement des suites d'une greffe d'organe offre un bon exemple de l'utilisation de ces différents traitements. Les greffes d'organes sont toujours suivies de traitements par des immunosuppresseurs dits conventionnels comme par exemple l'Endoxan, les corticostéroïdes, la cyclosporine ou le FK506, destinés à protéger le greffon de la réponse immune de l'hôte qui conduit au rejet. Si ces traitements s'avèrent relativement efficaces, comme en témoignent les progrès réalisés dans les greffes d'organes, ils n'arrivent pas toujours à empêcher le rejet des greffes. D'autre part ces traitements ont deux autres inconvénients : (i) ils ne font que "geler" la réponse immune et doivent donc être poursuivis de façon permanente (l'arrêt complet des traitements immunosuppresseurs aboutit en général au rejet de l'organe même après plusieurs années) et (ii) ils sont totalement non spécifiques et donc à ce titre, inhibent aussi les réponses immunitaires favorables pendant toute la durée de ce traitement. Il en résulte une sensibilité accrue aux infections et à certains cancers. Cette situation compromet le pronostic vital des patients.

Lorsque des rejets non contrôlés par ces traitements immunosuppresseurs surviennent, il existe des options thérapeutiques en dehors d'une simple augmentation des doses des médicaments utilisés. C'est, par exemple, l'utilisation d'anticorps détruisant les lymphocytes T : sérums polyclonaux ou anticorps monoclonaux reconnaissant des molécules de surface exprimées à la surface des lymphocytes T (anti-CD3, anti-CD4). Ces traitements agissent en détruisant tous les lymphocytes T, que ce soit ceux responsables du rejet de greffe comme l'ensemble des autres lymphocytes T, et présentent donc également un défaut de spécificité, induisant un état d'immunodepression important chez les sujets.

Pour certaines pathologies de type auto-immun, la greffe de moelle osseuse ("GMO") autologue est apparue comme nouvelle approche thérapeutique. L'intérêt de ces traitements est de faire précéder la greffe de moelle osseuse d'un conditionnement aboutissant à une destruction de son système immunitaire, puis à greffer le malade, avec ses propres cellules hématopoïétiques, dans le but d'une reconstitution par des lymphocytes T naïfs. Cependant, comme ces lymphocytes sont autologues, ils pourront à nouveau être impliqués dans les processus immunopathologiques qui ont conduit à réaliser ce traitement. De plus, la reconstitution des lymphocytes T à partir des cellules de la moelle semble être, chez l'adulte, un processus très peu efficace.

La GMO allogénique est maintenant proposée pour certaines de ces pathologies comme les pathologies auto-immunes sévères, dans l'optique de reconstituer le patient avec des lymphocytes différents qui ne devraient pas participer aux réactions immunopathologiques. Cependant, la greffe de moelle osseuse allogénique est entachée d'une morbidité importante, due à la GVH, ce qui limite son utilisation. En effet, les lymphocytes T, présents dans le greffon hématopoïétique et réinjectés chez un patient immunodéprimé du fait du conditionnement nécessaire à la greffe vont être responsables d'une attaque dirigée contre les cellules de l'hôte appelé aussi réaction du greffon contre l'hôte (GVH). Cette réaction est parfois difficile à contrôler par les traitements immunosuppresseurs classiques et est même parfois mortelle.

Il n'est pas aujourd'hui légitime de proposer à un patient présentant des troubles articulaires chroniques, mais qui peuvent évoluer sur plusieurs années voire plusieurs dizaines d'années, un traitement ayant une mortalité supérieure à 10 %.

Le contrôle des réponses immunitaires pathologiques repose donc aujourd'hui essentiellement sur des traitements immunosuppresseurs non spécifiques qui ne font que "geler" transitoirement ces réponses. Malgré tout l'intérêt de ces traitements, qui ont largement prouvé leur efficacité en clinique, il est important de développer aujourd'hui d'autres modalités thérapeutiques dont l'objectif serait d'obtenir une immunosuppression plus spécifique et éventuellement permanente.
* Des documents décrivent l'administration de lymphocytes T modifiés pour comporter un gène suicide codant pour la thymidine kinase du virus herpès simplex (WO 97/45142 ; Contassot et al, 1998, Transplantation 65, 1365-70 ; Tiberghien, 1997, Hum. Gene Ther., 8, 615-624 ; Cohen et al, 1997, Blood, 89, 4636-4645 ; Tiberghien, 1997, Transfusion clinique et biologique, 4, 275-80 ; Helene et al, 1997, Journal of Immunology, 158, 5079-82 ; Bordignon et al, 1995, Human Gene Therapy, 6, 813-9.
WO 96/05287 décrit une méthode de sélection de lymphocytes T avec une spécificité antigénique.

La présente invention représente une nouvelle approche thérapeutique pour la régulation de la réponse immune. La présente invention concerne plus particulièrement un nouveau concept de modulation de la réponse immune des lymphocytes T, notamment de la réponse lymphocytaire T immunopathologique. L'invention présente de nombreux avantages par rapport aux approches décrites dans l'art antérieur, notamment en terme de spécificité, de stabilité (durée) et de confort pour les sujets.

Un premier aspect de l'invention décrit plus particulièrement une méthode de modification du système immunitaire d'un sujet comprenant le remplacement des lymphocytes T de ce sujet. Plus particulièrement, cette méthode comprend la déplétion des lymphocytes T (ou de sous-populations de lymphocytes T) du sujet (sans déplétion des autres cellules hématopoïétiques, dont les cellules souches), suivie de l'administration d'une composition comprenant des lymphocytes T modifiés (ou des sous-populations de lymphocytes T), autologues, syngéniques ou allogéniques. Cette méthode est plus particulièrement destinée à contrôler les réponses immunopathologiques médiées par les lymphocytes T.

Un autre aspect particulier de l'invention décrit également une méthode de régulation des réponses immunopathologiques induites par les lymphocytes T, comprenant le remplacement de tout ou partie des lymphocytes T d'un sujet par une population de lymphocytes T modifiés.

L'invention décrit également à une méthode de traitement de pathologies provoquées par des lymphocytes T, comprenant le remplacement de tout ou partie des lymphocytes T d'un sujet par une composition comprenant des lymphocytes T modifiés.

L'invention concerne aussi l'utilisation d'une population de lymphocytes T pour la préparation d'une composition destinée au traitement d'une immunopathologie médiée par les lymphocytes T chez un sujet ayant subi une déplétion spécifique de tout ou partie de ses lymphocytes T sans déplétion de ses autres cellules.

L'invention concerne aussi la préparation d'une composition de lymphocytes T dont le répertoire a été modifié (enrichi en ou au contraire déplété de certaines spécificités antigéniques).

La présente invention décrit donc une nouvelle approche médicale, fondée sur le remplacement des lymphocytes T d'un sujet, et souvent désignée dans ce qui suit par l'expression "échange de lymphocytes T".

La présente invention repose en partie sur un nouveau concept de l'homéostasie des lymphocytes T. En particulier, l'invention repose sur la notion selon laquelle, chez l'adulte l'homéostasie des lymphocytes T se constitue essentiellement à partir de mouvements de populations de lymphocytes T matures, et non pas, comme on le pensait jusqu'à présent, à partir de la différentiation de cellules souches hématopoïétiques se différenciant en lymphocytes T via le thymus.

L'ensemble des données (i) sur la reconstitution de l'immunopoïèse T après greffe de moelle osseuse réalisée à partir de cellules souches hématopoïétiques immatures purifiées (déplétées en lymphocytes et/ou purifiées sur CD34), (ii) sur l'immunopoïése après greffe de moelle osseuse en fonction de l'âge, et enfin (iii) sur l'homéostasie des lymphocytes T au cours de l'infection par le VIH, supportent ce nouveau concept.

Ces données sont également cohérentes avec l'ensemble des données actuelles concernant l'homéostasie des lymphocytes T chez la souris. En particulier, il a été décrit qu'une souris thymectomisée plus de trois jours après la naissance vit de façon normale avec un nombre de lymphocytes T normal. En revanche, une souris thymectomisée avant J3 ne développe pas de lymphocytes T, mais si on lui réinjecte des lymphocytes T matures, ceux-ci se développent et la souris vit normalement avec un nombre normal de lymphocytes T.

Le principe de l'invention est donc particulièrement original car il propose que le remplacement des lymphocytes T d'un patient ne passe pas forcément par une greffe de moelle ou de cellules souches hématopoïétiques (précédée d'une aplasie médullaire (conditionnement)) mais peut être réalisé par une méthode moins invasive, objet de cette invention, qui est « l'échange de lymphocytes T ».

L'invention offre donc un premier avantage par rapport aux techniques antérieures, qui réside dans la reconstitution rapide de l'immunité. Ainsi, selon la présente invention, les lymphocytes T injectés à un patient permettent une expansion beaucoup plus rapide que la reconstitution à partir des lymphocytes T issus de la différenciation de cellules hématopoïétiques, ce qui aboutit à obtenir le remplacement complet (ou presque) des lymphocytes T du patient par un pool de lymphocytes T issus de ceux injectés.

L'invention présente un autre avantage important comparativement aux techniques antérieures, qui réside dans son caractère de spécificité. Ainsi, contrairement aux techniques classiques utilisant des immunosuppresseurs ou aux GMO, qui sont précédées d'aplasies médullaires complètes, la présente invention :
- présente une spécificité vis-à-vis de lymphocytes T immunopathologiques, et
- ne modifie qu'une portion seulement du système immunitaire d'un sujet.

Enfin, l'invention repose aussi sur la destruction de clones de lymphocytes T immunopathologiques, et possède donc également un caractère de stabilité dans la mesure où les clones sont supprimés de manière définitive.

La présente invention offre donc de nombreux avantages par rapport aux méthodes antérieures, et présente des applications multiples.

Les résultats décrits ci-avant indiquant l'existence d'un compartiment autonome et homéostatique contenant les lymphocytes T sont la base d'un principe général de traitement par des lymphocytes T matures, ledit traitement comprenant au moins 3 opérations, qui peuvent si besoin est être séparées dans le temps :
1) - un prélèvement de lymphocytes T autologues, syngéniques ou allogéniques, le donneur ayant des lymphocytes T présentant des caractéristiques d'immunité, d'antigénicité, d'âge ou autre que l'on souhaite conférer au receveur,
   - le cas échéant la transformation génétique des lymphocytes prélevés pour leur conférer une propriété particulière,
   - le cas échéant la modification (enrichissement, déplétion, appauvrissement...) de cette population de lymphocytes en une sous-classe antigénique particulière, par déplétion, enrichissement ou appauvrissement in vitro ou ex vivo par exemple par immuno-affinité,
2) - la destruction de tout ou partie des lymphocytes T du patient receveur,
3) - l'injection desdits lymphocytes enrichis, déplétés, transformés ou non, chez le receveur après destruction de tout ou partie de ses propres lymphocytes.

La population des lymphocytes du donneur le cas échéant transformée génétiquement ou enrichie par déplétion d'une ou des sous-classes non recherchées peut être administrée au receveur

De la même façon, la destruction de tout ou partie des lymphocytes T du patient n'est pas nécessairement requise dans toutes les applications du procédé.

Les applications de ce type de traitement sont nombreuses et recouvrent toutes les pathologies ou altérations physiologiques impliquant le système immunitaire. En particulier, on peut appliquer ce type de traitement aux maladies auto-immunes, aux allergies, au vieillissement des fonctions immunitaires comme les anergies, etc.

Cette invention présente au moins deux spécificités :
- le remplacement total ou partiel des cellules T d'un patient,
- la destruction totale ou partielle de clones pathologiques que ceux ci soient responsables de la GVH et/ou d'une pathologie pré existante comme l'auto immunité et/ou plus généralement présentent une mauvaise régulation de la réponse immunitaire. Cette destruction est réalisée par un système qui détruit spécifiquement les clones activés des cellules réinjectées au moment choisi. Cette destruction peut être réalisée par tout moyen choisi par l'homme du métier, comme détaillé ci-après.

Plus spécifiquement, un premier objet de l'invention concerne l'utilisation d'une population de lymphocytes T, pour la préparation d'une composition destinée à être administrée à un sujet ayant subi une déplétion de ses lymphocytes T, sans déplétion de ses autres cellules hématopöiétiques, dont les cellules souches.

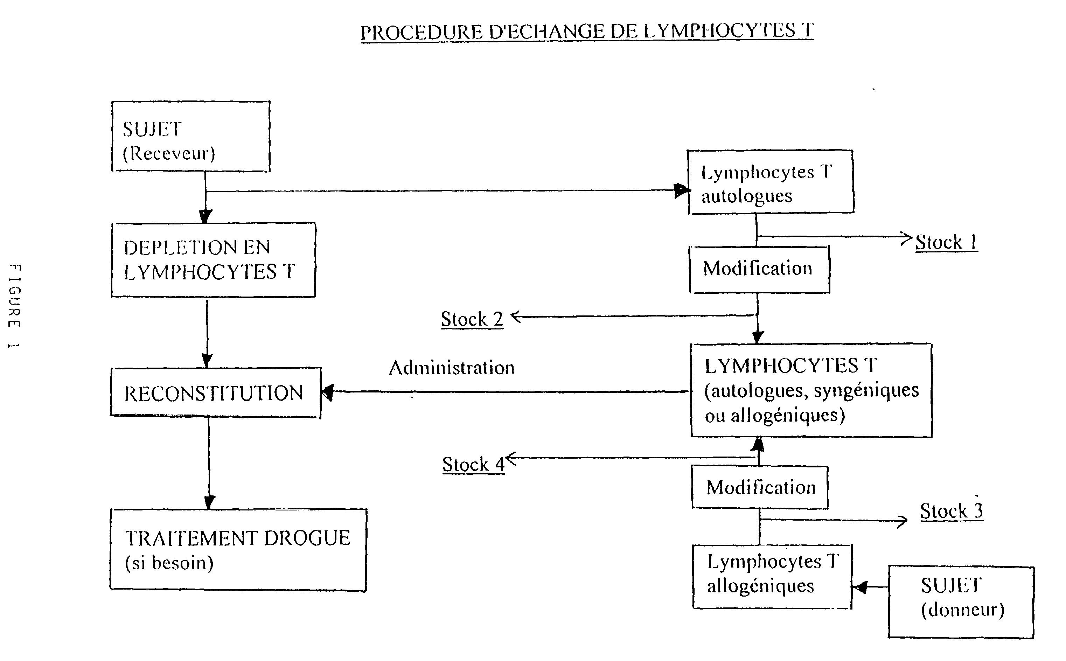

La présente invention repose donc, d'une part sur la déplétion des lymphocytes T du receveur, d'autre part sur l'administration d'une composition de lymphocytes T. Un schéma général de cet "échange de lymphocytes T" est représenté sur la Figure 1.

Comme il ressort de cette figure, la population de lymphocytes T utilisée peut comprendre des lymphocytes T autologues, syngéniques, ou allogéniques vis-à-vis du sujet.

Le terme "autologue" désigne une population de cellules provenant du sujet auquel elles sont administrées. Dans ce mode de mise en oeuvre, les cellules T sont donc prélevées du sujet avant la réalisation de la déplétion.

Le terme "syngénique" désigne une population de cellules provenant d'un jumeau identique au sujet auquel elles sont administrées.

Le terme "allogénique" désigne une population de cellules provenant d'un autre sujet de la même espèce (apparentée ou non), c'est-à-dire d'un autre être humain dans le cas du traitement d'un patient humain.

La population de lymphocytes T peut être obtenue et préparée par toute technique connue de l'homme du métier. Ainsi, les lymphocytes peuvent être soit isolés du sang, des organes lymphoïdes ou autre chez un sujet par les techniques classiques (cytaphérèse, gradients de densité, Tri cellulaire, etc.) soit obtenus à partir de banques stockées. Pour un prélèvement chez un sujet, les lymphocytes sont préférentiellement obtenus à partir des cellules mononuclées du sang périphérique (PBMC) ou du sang du cordon ombilical. Le sang de cordon ombilical est une source intéressante. En effet, les lymphocytes T du cordon ombilical présentent l'avantage de comprendre un répertoire très diversifié, et de pouvoir supporter de très nombreuses divisions.

S'agissant d'une population de lymphocytes T autologues, ils peuvent donc, dans un mode de mise en oeuvre particulier de l'invention, être prélevés du sujet lui-même (ou de son jumeau vrai s'il existe) par cytaphérèse à partir des PBMC ou du sang ombilical.

S'agissant d'une population de lymphocytes T allogéniques, ils peuvent, dans un mode de mise en oeuvre particulier de l'invention, être prélevés à partir de tout donneur allogénique, choisi de préférence pour présenter des compatibilités HLA avec le receveur, de sorte qu'il puisse y avoir coopération entre les cellules présentatrices d'antigènes du receveur et les lymphocytes T du donneur. Dans certaines situations particulières, les lymphocytes T pourront être prélevés à partir d'un donneur choisi pour une caractéristique particulière (par exemple sa capacité prouvée de répondre à un agent infectieux).

Pour la mise en oeuvre de l'échange de lymphocytes T selon l'invention, il est préférable que la population de lymphocytes T utilisée comprennent des lymphocytes T représentant un répertoire diversifié. Ceci permet en effet de favoriser une reconstitution rapide de l'immunité du sujet, et ainsi d'éviter la persistance d'une immunodépression importante chez le sujet. A cet égard, on estime que le sang périphérique renferme environ 2 % de la totalité des lymphocytes T matures d'un sujet. Par ailleurs, on considère que pour un antigène spécifique donné, la fréquence des clones des lymphocytes T spécifiques est d'environ une cellule sur 10⁵. Ainsi, à partir d'environ 10⁸ lymphocytes T du sang périphérique, on a une population de lymphocytes T pour laquelle chaque spécificité antigénique est représentée par environ 1000 cellules. Une population de cette taille permet donc une représentation tout à fait satisfaisante du répertoire des lymphocytes T. Préférentiellement, lors du prélèvement, il est souhaitable d'obtenir une quantité de lymphocytes T de l'ordre de 10⁶ à 10⁹, préférentiellement 10⁷ à 10⁸ cellules environ, de manière à couvrir un répertoire immunologique suffisant. Ensuite, ces cellules peuvent être, si besoin est, cultivées et développées in vitro (ex vivo). Préférentiellement, la population de lymphocytes T utilisées pour l'administration comprend environ 10⁷ à 10¹² cellules T, encore plus préférentiellement entre 10⁸ et 10¹¹, avantageusement entre 10⁹ et 10¹⁰ cellules T. Il est entendu que ces quantités peuvent être adaptées par l'homme du métier.

Par ailleurs, la population de lymphocytes T (Ly-T) utilisée peut comprendre certaines cellules de nature différente (non Ly-T). Ainsi, avantageusement, la population de lymphocytes T comprend au moins 60% de Ly-T, de préférence au moins 80%, encore plus préférentiellement au moins 95% de Ly-T. Les autres types cellulaires présents peuvent être par exemple des lymphocytes B, ou d'autres cellules sanguines, généralement hématopoiétiques. La qualité de la population de lymphocytes T peut être déterminée par toute technique connue de l'homme du métier, et notamment par marquage avec différents anticorps spécifiques, et analyse par cytométrie du flux. Par ailleurs, la qualité des lymphocytes peut également être vérifiée par analyse de leur répertoire par cytométrie de flux ou par la technique de l'immunoscope.

Dans le cadre de la présente invention, la population de lymphocytes utilisée est généralement une population modifiée pour améliorer les propriétés immunitaires du sujet. La ou les modifications apportées aux cellules T peuvent être de natures diverses.

Ainsi, il peut s'agir de modification(s) d'ordre génétique ou immunologique par exemple.

Dans une première variante particulière de l'invention, la population de lymphocytes T utilisée comprend des lymphocytes T modifiés génétiquement.

Le terme "modifié génétiquement" indique que les lymphocytes comprennent un acide nucléique non naturellement présent dans des lymphocytes à l'état non modifié, ou un acide nucléique présent à un état non-naturel dans les lymphocytes (une séquence artificiellement amplifiée par exemple). Dans ce qui suit, cet acide nucléique sera désigné acide nucléique hétérologue.

Plus préférentiellement, les lymphocytes génétiquement modifiés utilisés dans le cadre de la présente invention comprennent un acide nucléique hétérologue comprenant un gène suicide. On entend plus particulièrement au sens de l'invention par le terme "gène suicide" tout acide nucléique codant pour un produit toxique, c'est-à-dire pour un produit (ARN, protéine, etc.) susceptible d'induire la destruction de la cellule qui le contient, par tout mécanisme. Préférentiellement, il s'agit d'un acide nucléique codant pour un produit (par exemple une protéine) ayant une toxicité conditionnelle, c'est-à-dire capable de transformer une drogue normalement inactive en un métabolite hautement toxique pour la cellule. Dans ce type de toxicité, bien que toutes les cellules modifiées génétiquement expriment le gène suicide, la toxicité cellulaire est contrôlée strictement par l'administration de la drogue.

Dans un mode de mise en oeuvre particulièrement préféré, l'acide nucléique utilisé (i.e., le gène suicide) est un acide nucléique dont l'action toxique atteint spécifiquement les cellules en division. Des exemples de ce type d'acides nucléiques sont notamment la thymidine kinase (TK).

Le gène suicide le plus utilisé, et qui semble avoir les propriétés biologiques les plus intéressantes pour le contrôle des réponses immunes cellulaires, code pour la thymidine kinase du virus *Herpes simplex* de type 1 (HSV1-TK). Cette enzyme, contrairement aux thymidines kinases cellulaires, est capable de phosphoryler différents analogues nucléosidiques tels que l'aciclovir (ACV) ou le ganciclovir (GCV) en dérivés monophosphates qui seront ensuite transformés en composés di- et triphosphates par les enzymes cellulaires. Ces composés triphosphates peuvent être ensuite incorporés par les polymérases cellulaires dans l'ADN en cours d'élongation. Cette incorporation induit une terminaison de l'élongation et déclenche l'apoptose de ces cellules [11-18]. Ainsi, les doses toxiques de GCV nécessaires pour détruire les cellules exprimant HSV1-TK sont au moins 100 fois inférieures à celles nécessaires pour détruire les cellules parentales (environ 10 fois pour l'ACV). Le GCV étant beaucoup plus efficace que l'ACV dans ce système, c'est la drogue la plus utilisée, bien qu'elle nécessite une administration par perfusion biquotidienne. L'induction de la mort cellulaire par blocage d'élongation de l'ADN implique que seules les cellules en division sont affectées par le GCV, ce qui a été confirmé expérimentalement. Ainsi, le gène HSV-1 TK a été utilisé pour la thérapie génique du cancer [5,6], et plus récemment pour la destruction des ly-T à l'origine de réponses immunes pathologiques [1,2,7-9].

Ce système possède donc trois propriétés particulièrement intéressantes pour la présente invention : (i) une toxicité conditionnelle dépendant de l'administration d'une drogue (un analogue de nucléoside) qui permettra de contrôler la période de destruction des ly-T ; (ii) une toxicité restreinte aux cellules en division, propriété des ly-T activés ; et (iii) une grande souplesse d'utilisation permettant différent schémas d'administration de la drogue.

Le prototype du gène suicide est le gène HSV1-TK fonctionnant avec le ganciclovir.

D'autres thymidines kinases peuvent également être utilisées, comme la thymidine kinase dérivée des virus herpès Equin de type IV. Ce peut être également des gènes TK tronqués comme le gène delta TK ou une enzyme d'origine humaine ayant été mutée de sorte à acquérir les propriétés de ces enzymes virales.

A cet égard, plusieurs équipes ont débuté des essais thérapeutiques fondés sur le transfert d'un gène HSV1-TK dans les Ly-T matures qui sont ensuite réinjectés au cours de traitement de leucémie par GMO allogénique [27,28]. Les premiers résultats connus de ces études indiquent qu'il est possible *in vivo* de contrôler la réactivité de ces ly-T par un traitement par le gancyclovir (GCV) [7]. Les expériences réalisées sur l'animal et sur l'homme montrent donc qu'il est possible de contrôler la GVH après greffe de moelle osseuse allogénique si les ly-T injectés sont porteurs du gène HSV1-TK. Cependant, le mode opératoire proposé reste extrêmement lourd car il comprend le remplacement de l'ensemble des lignées hématopoïétiques du receveur.

L'utilisation d'un acide nucléique codant pour une toxine conditionnelle constitue un mode de réalisation particulièrement préféré de l'invention. En effet, la présence de ce type de construction dans les lymphocytes utilisés pour l'administration permet de contrôler de manière spécifique les réponses T immunopathologiques. Ainsi, dans le cas de lymphocytes T autologues, la présence du gène suicide est utilisée pour détruire les lymphocytes T engagés dans les réponses immunes pathologiques et ainsi supprimer, de manière durable, les clones de lymphocytes responsables de ces pathologies. Dans le cas de lymphocytes T allogéniques utilisés dans le cadre d'une greffe de moelle osseuse, la présence du gène suicide est utilisée pour détruire les lymphocytes T engagés dans une réaction du greffon contre l'hôte, et ainsi éviter le développement de ce type de pathologies.

Ainsi, dans un exemple d'application de la présente invention, un patient atteint d'une pathologie médiée par une réponse T immunopathologique est soumis à une déplétion efficace en lymphocytes T (comme décrit plus loin), puis est reconstitué avec des lymphocytes T. Ces lymphocytes T peuvent être soit les lymphocytes T du patient lui-même (autologues) après qu'ils aient été transduits par un gène suicide (c'est-à-dire modifiés génétiquement), soit des lymphocytes T allogéniques, également modifiés. Les lymphocytes T matures ainsi injectés chez le sujet déplété de ses propres lymphocytes T matures vont très rapidement proliférer et s'expandre afin de reconstituer le pool des lymphocytes T matures suivant la propriété d'homéostasie des lymphocytes T matures de subir une expansion in vivo de telle sorte que leur nombre atteigne les valeurs avant déplétion. A cet égard, les rares Ly-T restant chez le sujet pourront d'autant moins se multiplier que le compartiment dédié aux Ly-T aura été "rempli" par les cellules injectées. Ensuite, lors de phases pathologiques, le sujet est traité par la drogue susceptible d'être transformée en métabolite toxique par le gène suicide utilisé, ce qui conduit à une élimination préférentielle des seuls lymphocytes T en division, c'est-à-dire des seuls lymphocytes T activés et donc responsables de la pathologie. Cet échange de lymphocytes T et le traitement subséquent en présence de la drogue permet donc de modifier in vivo et de manière durable (voire permanente) le répertoire lymphocytaire du sujet.

L'invention présente en outre l'avantage de pouvoir être mise en oeuvre même en l'absence de connaissance, a priori, des antigènes impliqués dans le développement de la pathologie concernée.

Selon une autre variante de l'invention, la population de lymphocytes T utilisée comprend des lymphocytes T modifiés immunologiquement. Plus particulièrement, cette modification immunologique comprend la modification in vitro (ou ex vivo) du répertoire des lymphocytes T de ladite population. De manière plus préférée, cette modification immunologique comprend la suppression in vitro ou ex vivo de clones de lymphocytes impliqués dans des réponses immunopathologiques et/ou de sous-populations de lymphocytes T ayant des propriétés immunologiques particulières (CD4+ ou CD8+, TH1 ou TH2...).

Avantageusement, la population de lymphocytes T utilisée comprend donc un "trou" dans le répertoire immunologique.

Ce mode de mise en oeuvre est particulièrement adapté aux situations dans lesquelles le ou les antigènes reconnus par les clones immunopathologiques sont connus. En effet, dans ce cas, il est possible de réaliser une déplétion in vitro ou ex vivo sur la population de lymphocytes T, par immunoaffinité au moyen desdits antigènes ou de fragments ou homologues de ceux-ci par exemple. Une telle déplétion peut être réalisée par exemple par passage de la population de lymphocytes T au contact d'un support sur lequel lesdits antigènes ou fragments ou homologues sont immobilisés. La déplétion des clones reconnaissant des antigènes spécifiques impliqués dans l'immunopathologie peut être également réalisée après un transfert de gènes suicides dans les lymphocytes T, si ceux-ci sont ensuite stimulés in vitro en présence de l'antigène et d'un traitement par le toxique conditionnel (GCV). En l'absence d'une modification génétique, ces clones de lymphocytes activés par l'antigène in vitro peuvent aussi être détruits par des anticorps couplés à des toxine reconnaissant spécifiquement des molécules exprimées par les lymphocytes T activés (récepteurs à l'II2).

Ce mode de mise en oeuvre peut être réalisé par exemple dans le cas de pathologies telles que la greffe d'organe dans lesquelles les alloantigènes du donneur d'organe sont impliqués.

Cette modification immunologique des lymphocytes T peut être mise en oeuvre aussi bien avec une population autologue ou syngénique qu'avec une population allogénique. En outre, elle peut également être combinée avec une modification génétique telle que décrite ci-dessus.

Ainsi, dans un mode particulier de mise en oeuvre, la population de lymphocytes T utilisée comprend des lymphocytes modifiés :
- immunologiquement, par déplétion de clones spécifiques et/ou de sous-populations de lymphocytes T ayant des propriétés immunologiques particulières (CD4+ ou CD8+, TH1 ou TH2...), et
- génétiquement par introduction d'un acide nucléique codant pour un produit toxique.

Selon une autre variante de l'invention, la population de lymphocytes T utilisée comprend des lymphocytes T allogéniques procurant une immunité accrue. En particulier, il est possible d'utiliser des lymphocytes T provenant d'un sujet présentant une réponse appropriée (prononcée ou accrue) à des agents infectieux. Ainsi, il est connu que la réponse immunitaire T est en partie contrôlée par des facteurs génétiques de grande diversité. De ce fait, vis-à-vis d'un antigène donné, certains sujets sont capables de montrer des réponses appropriées (bénéfiques) alors que d'autres ne le sont pas. Selon l'invention, il est ainsi possible de remplacer les lymphocytes T d'un sujet sensible à une pathologie donnée par ceux d'un sujet insensible.

Dans ce mode de réalisation, il n'est pas nécessaire que les lymphocytes T soient modifiés immunologiquement. Néanmoins, il est préférable, pour pouvoir contrôler toute réaction du greffon contre l'hôte, que ces lymphocytes T soient modifiés génétiquement comme décrit ci-avant.

Par ailleurs, dans un mode de réalisation particulier de l'invention, il est possible d'utiliser une population de lymphocytes T telle que décrite ci-dessus, modifiée génétiquement et/ou immunologiquement et/ou procurant une immunité accrue, comprenant en outre un acide nucléique codant pour un produit thérapeutique. En effet, selon la présente invention, il est également possible de conférer aux lymphocytes T utilisés une propriété nouvelle. Les lymphocytes T étant des cellules facilement prélevables, isolables et cultivables, elles peuvent en effet être utilisées simultanément comme source pour introduire (ou réintroduire) chez un sujet des protéines ou des fonctions absentes ou anormales.

Pour la modification génétique des lymphocytes T, plusieurs approches peuvent être utilisées, selon les techniques de l'homme du métier.

Dans un mode particulier de réalisation de l'invention, les lymphocytes sont modifiés génétiquement au moyen d'un vecteur viral. Dans ce mode de réalisation, l'acide nucléique hétérologue est par exemple introduit dans un vecteur viral, qui est ensuite utilisé pour infecter une population de lymphocytes T telle que décrite ci-avant.

Différents types de vecteurs viraux peuvent être utilisés, notamment des vecteurs rétroviraux ou AAV. Dans un mode préféré de mise en oeuvre, les lymphocytes sont modifiés génétiquement au moyen d'un vecteur rétroviral.

Les rétrovirus sont des vecteurs de choix pour le transfert de gènes dans les lymphocytes T car l'infection rétrovirale aboutit à l'intégration stable du génome dans les cellules. Ceci est une propriété très importante compte tenu du fait que l'expansion lymphocytaire, soit in vitro soit in vivo après injection chez le sujet, suppose que le transgène soit très stable au cours de la ségrégation pour être transmis à chaque division cellulaire. En ce qui concerne les types de rétrovirus pouvant être utilisés, on peut citer par exemple les rétrovirus appartenant à la famille des oncovirus, des lentivirus ou des spumavirus. Dans la famille des oncovirus, on peut citer notamment les oncovirus lents, non porteur d'oncogène, tels que par exemple MoMLV, ALV, BLV, ou MMTV et les oncovirus rapides, tels que RSV par exemple. Dans la famille des lentivirus, on peut citer par exemple HIV, SIV, FIV ou CAEV.

Des techniques de construction de rétrovirus recombinants défectifs ont été largement décrites dans la littérature (WO89/07150, WO90/02806, WO94/19478, etc.).

Dans un mode particulier de mise en oeuvre de l'invention, on utilise avantageusement un rétrovirus recombinant comprenant une enveloppe du virus GALV (rétrovirus pseudotypé avec GALV). En effet, il a été montré que l'infection des cellules hématopoiétiques par un rétrovirus recombinant est plus efficacement réalisée lorsque l'enveloppe rétrovirale est dérivée d'un rétrovirus appelé Gibbon Ape Leukemia Virus (GALV) (Movassagh et al., Hum. Gene Ther. 9 (1998) 225). A l'aide de cette enveloppe rétrovirale, nous avons par ailleurs montré qu'il était possible d'obtenir des taux de transduction supérieurs à 95 % dans les lymphocytes T matures avant toute sélection des cellules transduites (résultats non publiés).

D'autre part, le virus recombinant utilisé peut comporter des séquences régulatrices d'expression (promoteurs) spécifiques de certaines sous populations de lymphocytes T. Ainsi, dans certaines situations, il peut être souhaitable de n'exprimer l'acide nucléique que dans certaines populations de cellules T comme par exemple les cellules CD4+ ou CD8+, ou encore les cellules dites Th1 ou Th2 pour lesquelles des marqueurs spécifiques viennent d'être décrits chez la souris, permettant leur séparation. Ces cellules étant caractérisées par le type de cytokines qu'elles produisent (par exemple IL2 ou IFNg pour les Th1, et lL4 pour les Th2), il est possible d'utiliser les séquence régulatrices de ces différents gènes pour contrôler l'expression de l'acide nucléique hétérologue (notamment du gène suicide). Il est également possible d'utiliser des promoteurs contrôlant une expression spécifique dans telle ou telle population de lymphocytes T, comme par exemple le promoteur codant pour la molécule CD4.

Par ailleurs, dans un autre mode particulier de mise en oeuvre de l'invention, les lymphocytes sont génétiquement modifiés au moyen d'un rétrovirus produit dans une lignée d'encapsidation comprenant un gène pol tronqué.

L'un des risques associés à l'utilisation de vecteurs viraux pour le transfert de gènes est la possibilité de générer des virus réplicatifs. L'utilisation de lignées dites à génome séparé a permis de limiter ce phénomène. Il est possible de minimiser encore celui-ci en utilisant des constructions spécifiquement conçues pour diminuer le plus possible les événements de recombinaison. Nous avons par exemple montré que la troncation de l'extrémité 3' du gène Pol jusqu'au site de coupure par l'enzyme Sma1 sur le génome du rétrovirus de Moloney écotrope (PNCA) permet de conserver intégralement la fonction polymérase tout en supprimant l'ensemble des séquences chevauchant entre le gène Pol et le gène codant pour l'enveloppe des virus Moloney. L'utilisation de ce type de constructions pour réaliser les rétrovirus recombinants capables de transduire les lymphocytes T permet donc une amélioration en terme de sécurité.

L'infection des lymphocytes T par les vecteurs rétroviraux peut être réalisée selon toute technique connue de l'homme du métier (incubation avec un surnageant de rétrovirus, co-culture lymphocytes T-cellules d'encapsidation de rétrovirus, techniques Transwell, etc.). Une méthode particulièrement efficace a été décrite par Movassagh et al. (Cf Supra), comprenant une étape de centrifugation éventuellement répétée. Cette méthode peut être avantageusement mise en oeuvre pour la modification génétique des lymphocytes T selon l'invention.

Pour une meilleure mise en oeuvre de la présente invention, il est souhaitable d'obtenir un transfert de gènes le plus efficace possible dans les lymphocytes T. Ainsi, on préfère utiliser une population de lymphocytes T comprenant au moins 50%, de préférence au moins 65%, plus préférentiellement au moins 80% de lymphocytes T génétiquement modifiés. Dans un mode de réalisation idéal, on utilise une population de lymphocytes T dont le taux de modification génétique est le plus proche possible de 100 %.

Comme indiqué ci-avant, de tels niveaux peuvent être obtenus in vitro ou ex vivo, en utilisant par exemple une enveloppe GALV, et le cas échéant dans certaines conditions d'infection (Movassagh et al., supra). Par ailleurs, dans la mesure où la modification génétique est réalisée in vitro, il est également possible d'améliorer les niveaux de transduction en sélectionnant les cellules effectivement infectées. Pour cela, différentes techniques de sélection connues de l'homme du métier sont disponibles, et notamment l'utilisation d'anticorps reconnaissant des marqueurs spécifiques à la surface des cellules infectées, ou l'utilisation de gènes de résistance (comme le gène de résistance à la néomycine et la drogue G418 par exemple), ou encore de composés toxiques pour les cellules n'exprimant pas le transgène (i.e., la thymidine kinase). A cet égard, il pourrait être possible d'utiliser une molécule non toxique uniquement pour les cellules porteuses du gène HSV1-TK. La méthode pour trouver cette molécule est de réaliser un crible avec une bibliothèque de molécules connues pour leur toxicité et de tester celles qui sont 1) modifiées par l'enzyme HSV1-TK et 2) dont les métabolites ne sont plus toxiques. L'avantage d'une telle méthode est qu'il n'a pas besoin de transférer un deuxième gène et que la sélection ne porte que sur les cellules indésirables.

Préférentiellement, la sélection est réalisée en utilisant un gène marqueur, introduit dans le vecteur rétroviral, exprimant une protéine membranaire. La présence de cette protéine permet ainsi une sélection par les techniques classiques de séparation comme le tri par billes magnétiques, colonnes, ou le tri par cytomètre de flux. Dans cette optique, un gène comme celui codant pour la molécule Thy1 humaine est avantageux. On peut aussi envisager d'utiliser une molécule telle la molécule CD34, absente des Ly-T matures car il existe déjà des systèmes de sélections de cellules portant cette molécule de surface, ces systèmes ayant déjà été validés pour le tri de cellules et utilisés en clinique. En outre, ces marqueurs peuvent également comprendre des séquences étiquettes ("tag"), par exemple le tag c-myc.

L'utilisation d'un gène marqueur d'expression membranaire présente par ailleurs deux avantages supplémentaires dans le cadre de la présente invention : (i) il permet de suivre facilement les lymphocytes T génétiquement modifiés lorsqu'ils ont été injectés, et surtout (ii) de les détruire (même en l'absence de division) grâce à l'action d'un anticorps spécifiquement dirigé contre cette molécule (si cela s'avérait nécessaire).

Avantageusement, pour réaliser l'expression du gène marqueur (tel que Thy-1) il est préféré d'utiliser des vecteurs dits bicistroniques pour lesquels les gènes toxiques (e.g., HSV1-TK) et marqueur (e.g., Thy1) sont séparés par une séquence IRES.

La population de lymphocytes T obtenue, le cas échéant modifiée génétiquement et/ou immunologiquement et/ou ayant une immunité accrue, peut être conditionnée dans tout milieu et tout dispositif approprié. Ainsi, les milieux utilisables sont tous milieux de culture de cellules mammifères (RPMI, DMEM, etc.) ou toutes autres solutions adaptées à la conservation et/ou au stockage de cellules mammifères (solutions salines, tampons, etc). Le dispositif utilisé peut être par exemple un tube, flasque, boite, ampoule, seringue, poche, etc., de préférence en condition de stérilité appropriée à un usage pharmaceutique. La composition cellulaire peut être utilisée extemporanément ou stockée (au froid, congelée, ou lyophilisée, par exemple) en vue d'une utilisation ultérieure. Par ailleurs, comme indiqué plus loin, des banques de telles cellules peuvent avantageusement être réalisées, dans les conditions de préparation et stockage décrites ci-dessus.

Comme indiqué ci-avant, l'invention consiste en un échange de lymphocytes T d'un sujet, et comprend donc l'administration de lymphocytes T matures à un sujet ayant subi une déplétion de ses propres lymphocytes T.

Préalablement à l'administration, le sujet est donc soumis à une étape de déplétion de ses lymphocytes T existants. Bien entendu, lorsque la population de lymphocytes T utilisée est une population de lymphocytes T autologues, cette population est préparée préalablement à la déplétion.

La déplétion peut être effectuée de différentes manières. Ainsi, lorsqu'il y a une greffe de moelle osseuse concomitante, la déplétion peut être réalisée par le conditionnement, dont la radiothérapie.

Néanmoins, la déplétion est plus préférentiellement réalisée par traitement du sujet en présence d'un ou plusieurs agents immunosuppresseurs, en particulier en présence d'un agent immunossupresseur spécifique des lymphocytes T. De ce fait, le sujet n'est pas soumis à une aplasie totale de la moelle et conserve une certaine immunité, en dépit de l'immunosuppression des lymphocytes T.

Préférentiellement, l'agent immunossupresseur utilisé est un "sérum antilymphocytaire", ou un ou plusieurs anticorps monoclonaux spécifiques des molécules de surface des lymphocytes T. En particulier, il est possible d'utiliser un sérum ou des anticorps anti-CD3, CD4 et/ou CD8. Ce type d'immunosuppresseurs (sérum ou anticorps deplétants) a déjà été utilisé en thérapeutique. Ainsi, des expériences réalisées chez la souris et chez l'homme montrent qu'il est possible de dépléter très efficacement un animal ou un malade en lymphocytes T matures par ces traitements (Muller et al., Transplantation 64 (1997) 1432 ; Caillat-Zucman et al., Transplantation 49 (1990) 156).

Il n'est pas nécessaire que la déplétion en lymphocytes T matures soit complète pour que l'échange de lymphocytes T selon l'invention soit efficace. Ainsi, même si un certain pourcentage de lymphocytes T subsiste, il est peu probable que ceux-ci suffiront à re-induire l'immunopathologie. Généralement, les traitements immunosuppresseurs décrits ci-dessus conduisent à une déplétion supérieure à 90%, souvent à 95%, ce qui est tout à fait approprié à la présente invention.

La composition de lymphocytes T peut être administrée de différentes façons et selon différents protocoles. Il est souhaitable d'administrer les lymphocytes T à une date telle que les traitements administrés aux patients dans le but de détruire leurs propres lymphocytes T aient été éliminés, de sorte qu'ils ne puissent attaquer à leur tour les lymphocytes T qui vont être injectés.

Préférentiellement, la composition est administrée peu de temps après (par exemple moins de 48 heures après) la déplétion, de manière à favoriser l'expansion des lymphocytes T matures injectés, qui vont naturellement avoir tendance à combler le « trou » en lymphocytes matures. Par ailleurs, les traitements peuvent être répétés en cas de besoin, avec les mêmes cellules, voir des cellules de donneurs différents mais selon le même principe thérapeutique. L'administration peut être réalisée avantageusement par injection intraveineuse ou intraartérielle.

Comme indiqué ci-avant, les doses administrées sont généralement comprises entre 10⁸ et 10¹¹ lymphocytes T par sujet.

Par ailleurs, au cours des procédures décrites ci-avant pour la préparation des populations de lymphocytes T, il est possible de conserver des cellules du patient (normales et ou transduites par le gène suicide) qui peuvent servir en cas de besoin (stocks 1 et 2 sur la Figure 1). Par exemple, si des effets indésirables survenaient, il serait toujours possible de dépléter les cellules réinjectées (par exemple allogeniques) pour réinjecter alors les cellules (normales) du patient.

De même, dans le cas d'un échange allogénique, les cellules du donneur (transduites ou normales) peuvent être également conservées pour utilisation ultérieure si besoin (stocks 3 et 4 sur la Figure 1). Il est d'ailleurs possible que chez un patient ayant reçu des lymphocytes T allogéniques transduits dont on aura contrôlé la GVH, il soit alors possible d'injecter des lymphocytes normaux du même donneur sans qu'il y ait de GVH (tolérance active). Cela aurait l'avantage de réinjecter des cellules qui n'auraient pas été cultivées. De ce fait, avantageusement, le procédé de l'invention comprend également une étape de constitution de banques de cellules T, transduites ou non, issues du donneur et du receveur.

Suite à l'administration des compositions décrites ci-dessus, l'invention permet de contrôler l'apparition ou le développement d'immunopathologies, soit par administration de la drogue métabolisée en produit toxique (dans le cas de lymphocytes T modifiés génétiquement par un gène toxique), soit simplement par la constitution d'une immunité accrue chez le sujet.

Un autre objet de l'invention réside également dans un produit comprenant:
- un ou plusieurs agents immunosuppresseurs, et
- une composition comprenant une population de lymphocytes T telle que définie ci-avant,
en vue d'une utilisation séparée ou espacée dans le temps, notamment pour le contrôle des réponses immunopathologiques.

Préférentiellement, la composition comprend un répertoire diversifié de lymphocytes T.

Avantageusement, la composition comprend une population de lymphocytes T comprenant un gène suicide, et le produit de l'invention comprend en outre une drogue susceptible d'être transformée en métabolite toxique par le gène suicide.

L'invention est encore relative à un procédé de préparation d'une composition de lymphocytes T modifiés comprenant:
- le prélèvement de lymphocytes T chez un sujet, et
- la création d'un trou du répertoire dans ces lymphocytes ou plus généralement la modification sélective du répertoire.

Plus particulièrement, le trou du répertoire est réalisé par déplétion des lymphocytes T spécifiques d'antigènes impliqués dans des pathologies.

L'invention concerne aussi toute population de lymphocytes T prélevée chez un sujet et déplétée des clones de lymphocytes spécifiques d'un ou plusieurs antigènes impliqués dans une ou des pathologies.

Par ailleurs, l'invention peut également être mise en oeuvre avec tout autre type de cellule à compartiment, c'est-à-dire tout type de cellule répondant aux critères suivants :
- on peut les prélever, on peut détruire l'ensemble de la population et on peut les (re)injecter (avec modification) et elles prennent la place de celles que l'on a détruite.
- on peut envisager par exemple une sous-population de Ly-T ou d'autres cellules circulantes (cellules musculaire satellite).

Les applications de l'invention sont extrêmement nombreuses. Elles concernent notamment, aussi bien pour les lymphocytes T autologues que allogéniques modifiés selon l'invention, toutes les pathologies auto-immunes et inflammatoire chronique au sens large (polyarthrite rhumatoïde, sclérose en plaque, etc.), les infections virales (hépatites A, B, C, HIV, etc.) ou les maladies génétiques. Le remplacement des lymphocytes T concerne également les transplantations d'organes ou de cellules (allogéniques, voire xénogéniques) ainsi que toutes les pathologies pour lesquelles on peut souhaiter réinjecter des lymphocytes modifiés pour, par exemple, sécréter une protéine thérapeutique. Ce système peut également être avantageux pour pouvoir contrôler la réponse immunitaire contre une protéine thérapeutique administrée par exemple pour corriger un déficit génétique en cette protéine. Un des inconvénients dans cette situation est que, du fait du déficit génétique, le patient reconnaît cette protéine comme étrangère et la rejette par une réponse immunitaire. Selon les principes de l'invention, si les lymphocytes T expriment un gène suicide, il est alors possible de contrôler cette réponse immunitaire néfaste.

Ainsi, les maladies auto-immunes, dites maladies de système (ex: Lupus Erythémateux Disséminé, Polyarthrite Rhumatoïde, Polymyosites...) sont des affections ayant clairement une composante immunologique dont témoignent les investigations biologiques et histologiques réalisées chez les patients. Pour beaucoup de ces pathologies le *primum movens* n'est pas connu et il semble que l'origine de la pathologie soit multifactorielle. Néanmoins, l'élément central reste une réponse immunitaire inadaptée. De plus, dans ces pathologies, il est généralement possible de définir la période pendant laquelle les ly-T responsables de la pathologie sont activés et donc en division. De ce fait, l'administration de la prodrogue durant cette phase permet, selon la présente invention, d'affecter principalement ces cellules. De plus, cette stratégie aboutit à la délétion des clones de cellules responsables de la pathologie, et donc en principe à une immunosupression durable, voire permanente en l'absence de production centrale de ly-T.

Par exemple, dans un mode particulier de mise en oeuvre de l'invention, un patient souffrant de polyarthrite rhumatoïde reçoit, après déplétion de ses lymphocytes T propres, une administration d'une composition comprenant ses propres lymphocytes T modifiés génétiquement pour exprimer un gène suicide (composition autologue) ou des lymphocytes T allogéniques, modifiés ou non. Ensuite, lors de poussées éventuelles de polyarthrite rhumatoïde survenant après l'administration, un traitement par une drogue transformée en métabolite toxique par le gène suicide permet de détruire les clones réactifs et donc de supprimer la réponse immunopathologique.

La présente invention est également utilisable pour le traitement des immunopathologies viro-induites. La réponse immunitaire contre les agents infectieux peut souvent avoir des conséquences immunopathologiques allant parfois jusqu'à la mort. L'exemple le plus classique est celui de la réponse à certains virus responsables d'hépatites. Ces virus se répliquent dans les hépatocytes et c'est la destruction par le système immunitaire des hépatocytes infectés qui aboutit à l'hépatite, parfois mortelle. Dans d'autres situations, la réponse immunitaire de l'hôte semble incapable d'éradiquer le virus, et au contraire participe au maintien d'une hépatite chronique. Ceci est bien illustré par l'évolution des hépatites dites à virus C. Alors que deux tiers des patients environ sont capables de se débarrasser du virus, le tiers restant développe une hépatite chronique. L'évolution de cette hépatite chronique est indépendante du taux de réplication virale et est, au contraire, accompagnée de signes biologiques témoignant d'une réponse dysimmunitaire (par exemple présence fréquente d'anticorps anti-DNA ou d'une cryoglobulinémie). La présente invention permet d'éliminer le/les clones de lymphocytes T actifs responsables de l'immunopathologie, et donc de diminuer les conséquences de l'infection sur l'hôte.

D'autre part, la synthèse des IgE impliquées dans certaines allergies est également sous le contrôle des lymphocytes T. Par ailleurs, la production des lymphocytes B est réalisée à partir de cellules immatures de la moelle osseuse et ces cellules ont une demi-vie beaucoup plus courte que les lymphocytes T. De ce fait, il est aussi possible en utilisant la technique de l'échange de lymphocytes T selon l'invention de contrôler les réponses allergiques, et plus généralement les réponses anticorps en contrôlant les réponses T.

La présente invention est également utilisable pour le traitement ou la prévention du Rejet de greffes d'organe.

Le traitement classique d'un certain nombre de pathologies d'organes est, quand cela devient nécessaire, le remplacement de cet organe par un organe sain provenant d'un donneur décédé (ou d'un donneur vivant dans certains cas, voire à terme d'une autre espèce). Bien qu'un soin rigoureux soit apporté à sélectionner des donneurs d'organes présentant le maximum de compatibilité vis-à-vis des antigènes d'histocompatibilité, hormis les situations de greffes entre jumeaux homozygotes, la greffe d'organe conduit toujours au développement d'une réponse immunitaire dirigée contre des antigènes spécifiquement exprimés par cet organe. Malgré les traitements immunosuppresseurs mis en route, cette réaction aboutit souvent au rejet de l'organe greffé (c'est la principale cause d'échec des greffes allogéniques). Mis à part certains rejets suraigus ou aigus qui impliquent essentiellement des réponses humorales, la plupart du temps le rejet des greffes d'organes est essentiellement médié par des lymphocytes T.

De nombreuses équipes ont développé des projets de greffes de cellules allogéniques ou xénogéniques modifiées ou non, afin qu'elles produisent un facteur dans un but thérapeutique (ex cellules des îlots β du pancréas, fibroblastes ...). En particulier, ceci a été proposé dans des maladies aussi variées que le diabète, la maladie de Parkinson, voire même en thérapie génique dans les organoïdes. Le principal obstacle à de telles greffes reste le rejet des cellules allo ou xénogéniques. Pour pallier cet inconvénient, de très nombreux dispositifs ont été proposés afin de séparer les cellules greffées du système immunitaire. Ces systèmes vont de micro-encapsulation à l'insertion des cellules dans des matériaux poreux ou semi-perméables, etc. Malheureusement, aucun de ces systèmes n'a fait preuve d'une efficacité suffisante pour pouvoir être proposé en clinique.

Ces malades étant, par ailleurs, en bonne santé immunologique, il est tout à fait licite d'envisager l'échange standard de lymphocyte T tout en conservant un stock des lymphocytes des malades au cas où un éventuel problème apparaîtrait.

La présente invention offre désormais une nouvelle approche médicale pour favoriser le succès des greffes d'organes ou de cellules. En particulier, un échange de lymphocytes T est pratiqué chez le sujet receveur, ce qui permet, si besoin est, de contrôler tout développement d'une réponse immune éventuelle dirigée contre le greffon, par destruction sélective des lymphocytes T alloréactifs. Dans le cadre des greffes d'organes ou de cellules, il est possible d'appliquer ces thérapeutiques, soit en réimplantant chez le receveur un organe ou des cellules et des lymphocytes T allogéniques provenant du même donneur, (et qui sont donc tolérant à l'organe ou aux cellules transplantés), soit de transplanter un organe ou des cellules d'un donneur allogénique et de réimplanter au patient ses propres lymphocytes T génétiquement modifiés afin de contrôler la réponse contre cet organe ou ces cellules. Enfin, il est possible d'envisager de greffer un organe ou des cellules et des lymphocytes T allogéniques provenant de deux donneurs distincts.

Un mode de mise en oeuvre particulièrement avantageux de la présente invention réside dans le traitement des immunopathologies dans le cadre de greffe d'organes ou de cellules. Plus particulièrement, le traitement désigne la prévention, totale ou partielle, ou la réduction ou la suppression des immunopathologies médiées par les lymphocytes T, qui sont responsables des échecs de greffe.

De manière similaire, l'invention peut également être mise en oeuvre pour le traitement (la prévention, la réduction ou la suppression totale, par exemple) de la maladie du greffon contre l'hôte (GVHD). La greffe de moelle osseuse est un traitement classique dans un grand nombre de situations cliniques, notamment pour beaucoup de leucémies. Le traitement conventionnel repose alors sur le conditionnement du donneur, dont le but est d'éliminer le maximum de cellules tumorales, et qui a pour conséquence de créer une aplasie médullaire nécessitant une greffe de moelle osseuse. Cette greffe de moelle osseuse est réalisée de préférence avec des cellules allogéniques car celles-ci ont fait la preuve qu'elles peuvent être responsables d'un effet dit du « greffon contre la leucémie » qui limite de façon considérable le taux des rechutes des leucémies ainsi traitées. Malheureusement, les cellules T allogéniques sont aussi responsables de la maladie du greffon contre l'hôte toujours grave et parfois mortelle. L'utilisation de la présente invention permet de détruire les cellules T responsables de la GVH, et donc de traiter cette immunopathologie.

D'autres exemples de pathologies pouvant être traitées par remplacement de lymphocytes T sont notamment les infections par le VIH ou certaines maladies génétiques.

Ainsi, les lymphocytes T sont une cible principale de l'infection par le VIH dont l'évolution clinique dépend en grande partie du taux de ces lymphocytes chez le patient infecté. Certaines stratégies thérapeutiques de l'infection par le VIH sont fondées sur le transfert de lymphocytes T génétiquement modifiés pour résister au virus. Cependant il existe aussi des patients dont les lymphocytes T sont moins susceptibles à l'infection par le VIH du fait de mutations spontanées dans des gènes codant pour des protéines impliquées dans le cycle réplicatif du virus. Ainsi, comme pour la réponse au virus de l'hépatite, il existe des différences génétiques exprimées par les lymphocytes T qui les rendent plus ou moins sensibles à une infection par le VIH. La présente invention permet de mettre à profit ces facteurs génétiques de grande diversité, notamment par le remplacement des lymphocytes T d'un sujet sensible à une pathologie donnée par ceux d'un sujet insensible.

Il existe d'autre part un grand nombre de maladies génétiques qui sont liées à l'incapacité de synthétiser une protéine qui, si elle est synthétisée par certains types cellulaires spécifiques (pas nécessairement les lymphocytes T) a néanmoins une action générale. Ainsi, ces pathologies peuvent-elles être souvent traitées par greffe de moelle osseuse lorsqu'un donneur compatible peut être trouvé. Ces mêmes maladies pourraient être traitées par transfert de lymphocytes T (muccopolysaccharidose..).

Il existe également des maladies génétiques affectant spécifiquement les lymphocytes (déficit en Adénosine Déaminase,...), qui peuvent aussi être traitées par l'échange standard de lymphocytes T selon l'invention. Enfin, les possibles accidents d'irradiation ou toute autre insuffisance médullaire aiguë (par exemple toxique, médicamenteuse...) peuvent aussi être pris en charge par un échange de lymphocytes T selon l'invention. On peut ainsi constituer des banques de cellules souches hématopoïétiques et de banques de lymphocytes T provenant des mêmes donneurs, pour lesquels seuls les lymphocytes T auraient subi une modification génétique permettant le contrôle des réactions immunopathologiques. Ainsi, on dispose alors de "banques universelles" permettant les greffes de moelle avec lymphocytes T matures pour les situations le nécessitant.

Selon une autre variante de l'invention, la population de lymphocytes T du donneur peut être utilisée dans la prévention ou le traitement du vieillissement des fonctions immunitaires comme les anergies.

La présente invention sera décrite plus en détails à l'aide des exemples qui suivent, qui doivent être considérés comme illustratifs et non limitatifs.

### LEGENDE DES FIGURES

**Figure 1:** Représentation schématique du principe de l'Echange Standard de Lymphocytes T.

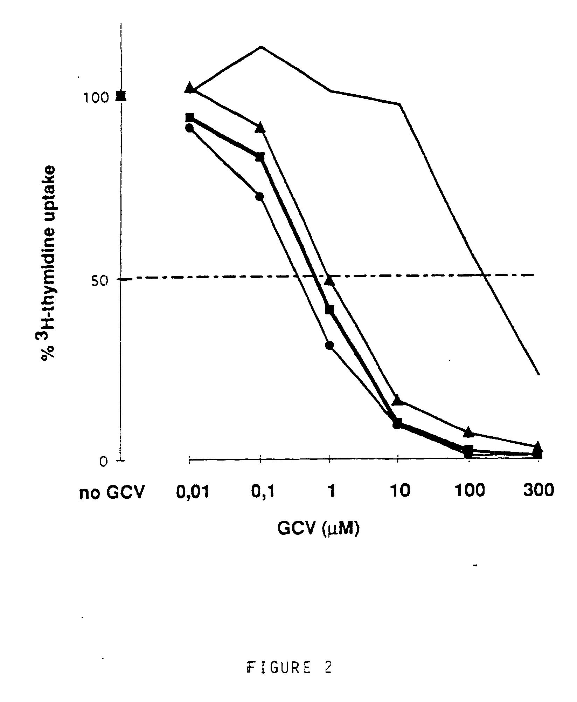

**Figure 2:** Sensibilité au GCV de cellules T exprimant une thymidine kinase. Les splénocytes ont été cultivés 2 jours en présence de ConA et de concentrations croissantes de GCV, puis chargées pendant la nuit avec la TdR tritiée (un "uptake" de 100% est celui observé en l'absence de GCV). Les triangles, carrés et ronds correspondent à différentes formes de TK. La courbe supérieure correspond aux lymphocytes T non transduits.

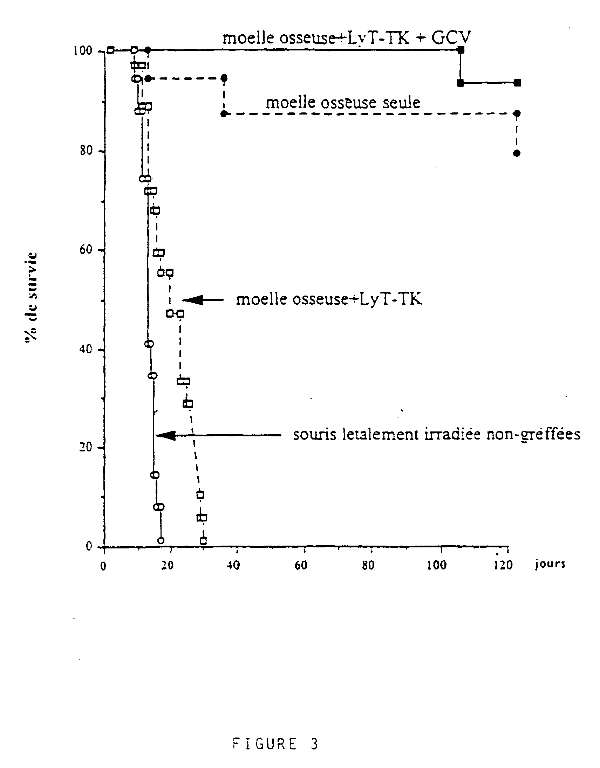

**Figure 3**: Effet protecteur de la GVH d'un traitement de 7 jours par le GCV. Le groupe contrôle de l'irradiation est constitué de souris irradiées non greffées (n=15=. Une GVH mortelle à J35 pour 100% des souris est induite par la co-injection de cellules de la moelle osseuse et de Ly-T allogéniques (n=25). La survie des souris traitées (n=13) est comparée à celle obtenue sur des souris ne recevant que de la moelle et ne développant donc pas de GVH (n=15).

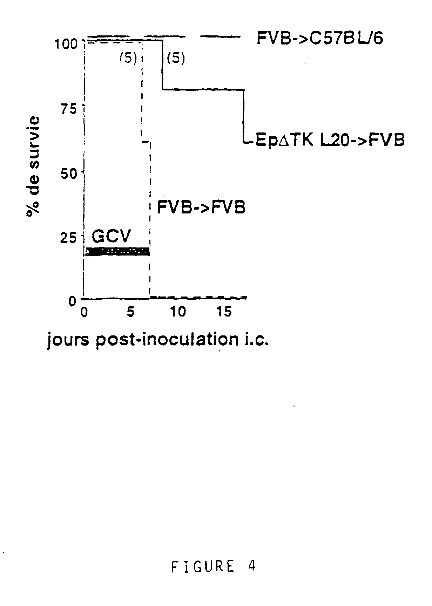

**Figure 4:** Contrôle pharmacogénétique de la chorioméningite lymphocytaire chez des souris exprimant le gène suicide dans les populations de lymphocytes CD4 et CD8. Des souris FVB ou C57BL/6 ont été irradiées et reconstituées par de la moelle osseuse syngénique provenant de souris FVB ou de souris transgéniques EPDTKL20, ou par de la moelle allogénique. Après reconstitution du système immunitaire les receveurs ont été infectés par voie intracérébrale par le virus de la chorioméningite lymphocytaire (10⁴ pfu de LCMV souche Arm/53b) et traitées par le GCV pendant 7 jours (injections biquotidiennes par voie intrapéritonéale de 100 mg/kg/j). Les souris exprimant le gène TK dans leurs lymphocytes sont protégées, survivent, ont éliminé le LCMV et possèdent un haut titre d'anticorps anti-LCMV.

### EXEMPLES

### 1. Etude de la Régulation de la réponse immunopathologique

Afin d'étudier la possibilité de contrôler des réponses immunitaires pathologiques à l'aide de gènes suicides, nous avons développé des souris transgéniques exprimant le gène HSV1-TK dans les ly-T. Cette expression est obtenue à l'aide des séquences régulatrices dérivées du gène codant pour la molécule CD4, dont nous avions montré préalablement qu'elles conduisaient à l'expression spécifique d'un transgène dans les lymphocytes T CD4⁺ et CD8⁺ matures, à l'exclusion des thymocytes immatures CD4⁻ CD8⁻ ou CD4⁺CD8⁺ [26]. *In vitro* les ly-T de ces souris transgéniques sont détruits efficacement par le GCV (Figure **2**).

Dans tous les exemples qui suivent, les lymphocytes T proviennent de souris transgéniques uniquement par souci de simplification de la procédure expérimentale. Les mêmes expériences peuvent être reproduites en prenant des lymphocytes T de souris ou humains dans lesquels le même gène suicide peut être transféré par un vecteur rétroviral selon les règles de l'art.

### 1.1. Contrôle de la GVH chez la souris

En utilisant des souris transgéniques exprimant le gène HSV1-TK dans les lymphocytes T, nous avons montré qu'il était parfaitement possible de contrôler la réponse allogénique responsable de la réaction du greffon contre l'hôte chez ces animaux. Nous avons donc ainsi fait la preuve de principe de cette capacité à contrôler les lymphocytes T responsables de la GVH. Il faut d'ailleurs mentionner que cette efficacité thérapeutique peut être obtenue par des traitements par le ganciclovir extrêmement courts, et que nous avons montré qu'à l'issue de ces traitements non seulement la pathologie était évitée, mais que la souris avait une réponse immunitaire normale.

Nous avons utilisé ces souris transgéniques comme donneuses de ly-T HSV1-TK⁺ pour la mise au point d'un modèle de maladie du greffon contre l'hôte (GVH) après greffe de moelle osseuse (GMO) allogénique [8]. Ainsi, chez la souris irradiée létalement, une GMO allogénique déplétée en ly-T matures aboutit à une survie de la totalité des animaux et à une reconstitution hématopoïétique complète par les cellules du donneur. En revanche, le greffon supplémenté en ly-T aboutit à une mortalité de 100% des animaux qui présentent les signes cliniques et histologiques de GVH. Dans ce modèle, nous avons montré que si les ly-T expriment le gène suicide HSV1-TK, un traitement par le GCV institué le jour de la greffe et prolongé pendant 7 jours permet la prévention de la GVH clinique (Figure 3). Lorsque ces animaux sont sacrifiés, l'analyse histologique objective l'absence de tout signe histologique de GVH chez ces animaux. Le traitement est également efficace sur une GVH déclarée.

Les cellules d'origine hématopoïétiques présentes chez ces animaux proviennent du donneur. De façon intéressante, nous avons observé que les ly-T qui ont survécu au traitement par le GCV sont toujours capables de répondre *in vitro* à des stimulations par un mitogène ou un tiers alloantigène, mais pas par des cellules provenant du donneur ou du receveur. Ces résultats ont montré qu'un traitement par le GCV permet d'éliminer les clones de ly-T alloréactifs, tout en préservant un compartiment de ly-T capables de répondre à d'autres stimulations antigéniques.

### 1.2. Traitement du diabète autoimmun chez la souris diabétique (NOD) par échange de lymphocytes T : preuve de principe

Cette expérience a pour but de démontrer l'efficacité de l'échange de lymphocytes T génétiquement modifiés sur le contrôle d'une maladie auto-immune (MAI) spontanée représentée ici par le modèle de la souris diabétique auto-immune NOD. L'échange de lymphocytes T est conduit dans des conditions proches de celles envisagées en clinique. L'échange de lymphocytes T se fait avec des lymphocytes T allogéniques au receveur permettant la destruction d'éventuelles cellules autoimmunes résiduelles. L'échange de lymphocytes T se fait avec des lymphocytes T génétiquement modifiés exprimant le gène suicide TK. Dans ce modèle, le contrôle de la GVH repose sur l'utilisation de lymphocytes T génétiquement modifiés exprimant le gène TK associé à un traitement par le ganciclovir (GCV). Ce système permet la destruction des lymphocytes T TK en division contrôlant ainsi la GVH. L'échange de lymphocytes T se fait après un conditionnement non myélo-ablatif des souris NOD par une irradiation de 8-9 Gy aboutissant à 100 % de survie à J 120 chez de telles souris. L'échange de lymphocytes T se fait par injection dans le sinus rétro-orbital des souris irradiées de 10⁷ cellules associé à un traitement préventif de 7 jours par le ganciclovir, initié au moment même de la greffe. Un premier groupe contrôle est constitué de souris recevant 10⁷ cellules de moelle osseuse allogénique mais pas de lymphocytes T. Chez ces souris, le greffon peut être partiellement ou totalement rejeté et la MAI peut réapparaître. Un deuxième groupe contrôle est constitué de souris recevant 10⁷ cellules de moelle osseuse provenant de souris NOD mais pas de lymphocytes T.

L'efficacité de la stratégie est évaluée par :
- l'analyse de la présence de lymphocytes T génétiquement modifiés caractérisés par l'expression de molécules du CMH d'origine du donneur;
- la détermination de signes cliniques du diabète autoimmun (glycémie supérieure à 2g/l) ;
- par la présence d'infiltrats lymphocytaires sur les îlots de Langerhans.

### RESULTATS

### • Origine des CSMNSP

**Tableau 1:**

| Etude du chimérisme 180 jours après la greffe. | | |
|---|---|---|
| | | Lymphocytes T d'origine du donneur |
| Contrôle NOD | 2 | 0/2 |
| NOD irrad. sublétalement | 3 | 0/3 |
| NOD greffées syng. | 6 | 0/6 |
| NOD greffées et protégées | 3 | 3/3 |

180 jours après la greffe, la reconstitution hématopoïétique est évaluée après marquage des CSMNSP par des Ac monoclonaux et analysée par cytométrie de flux. Les cellules B et T d'origine du donneur sont déterminées par l'expression de molécules de classe I du CMH (H-2^{q}). Les cellules B et T du receveur sont déterminées par l'expression de molécules de classe Il du CMH (J-A^{k}).

Chez les souris NOD chez lesquelles un échange de lymphocytes T a été réalisé, seules des cellules d'origine du donneur sont présentes.

### • Présence d'infiltrats lymphocytaires après échange de lymphocytes T

**Tableau 2 :**

| Péri-insulite et insulite chez la souris NOD après échange de Lymphocytes T. | | |
|---|---|---|
| | n | Présence d'infiltrats lymphocytaires Langerhansiens |
| NOD greffées syng. | 4 | 4/4 |
| NOD greffées et protégées | 3 | 0/3 |

180 jours après la greffe, le pancréas des souris est prélevé, fixé dans du liquide de Bouin puis placé dans de la paraffine. La présence d'infiltrats lymphocytaires est évaluée sur des coupes de 4 microns après marquage par l'hématoxiline et l'éosine.

Sur aucun pancréas de souris ayant subi un échange de lymphocytes T il n'a été observé d'infiltrat lymphocytaire.

Par ailleurs, les souris sont restées normoglycémiques attestant ainsi de l'absence de diabète.

En conclusion, les souris receveuses sont reconstituées par les lymphocytes T allogéniques et ne développent plus la maladie autoimmune.

### 1.3. Contrôle d'une immunopathologie viro-induite

Nous avons voulu appliquer le même système au contrôle d'une réaction d'immunopathologie induite par un virus. Le système expérimental le plus classique est celui du contrôle de l'encéphalopathie induite par le virus de la CML chez la souris.

La réponse immunitaire au virus de la CML est très bien connue. Ainsi, les animaux inoculés par voie intracérébrale avec ce virus meurent très rapidement d'une atteinte cérébrale rapidement fatale médiée par les lymphocytes T cytotoxiques. Chez les animaux ayant reçu une immunisation préalable, la cinétique de réactivité des lymphocytes T sensibilisés est accélérée conduisant à la protection et à la survie des souris. Chez les animaux recevant du ganciclovir au moment de l'injection intracérébrale, la destruction des lymphocytes T cytoxiques activés par la réplication virale aboutit à un contrôle de l'atteinte cérébrale (figure 4).

### 1.4. Contrôle d'un rejet de greffe

Nous avons ensuite utilisé nos souris pour essayer de contrôler les réponses d'immunité responsables du rejet d'un greffon d'organe. Le modèle utilisé est celui qui peut être considéré comme le plus physiologique. Il s'agit d'une greffe de coeur vascularisé en position hétérotopique chez la souris. Lorsque cette greffe allogénique est réalisée chez les souris non traitées, le rejet survient de façon régulière dans les quelques jours qui suivent la greffe. Lorsque les souris sont traitées par le ganciclovir sur une très courte période (7 jours à partir du jour de la greffe) le coeur est toléré de façon permanente et garde ses fonctions vitales comme en témoignent ses battements.

### 2. Protocole thérapeutique de l'Echange de Lymphocytes T appliqué aux pathologies auto immunes.

Le traitement débute par une ablation des lymphocytes T. Cette ablation peut être réalisée par exemple en combinant l'administration de "sérum antilymphocytaire" (immunoglobulines capables de reconnaître des lymphocytes T et de les détruire en présence du complément du patient qui sont habituellement produites chez le lapin par exemple par les laboratoires Mérieux). La quantité de ce SAL qui est administrée et la durée de l'administration sont dépendantes des données cliniques sur le lot pharmaceutique et des recommandations du fabricant. Ce traitement peut être réalisé en même temps que l'administration d'autres immunosuppresseurs comme le cyclophosphamide et la cyclosporine. Enfin, afin d'atteindre les lymphocytes T tissulaires qui pourraient être plus résistants à ces traitements, il est possible d'adjoindre une irradiation délivrée tout en protégeant la moelle osseuse du patient.

A l'issue de ce traitement, la délétion des lymphocytes T peut être analysée au niveau du sang périphérique par leur numération classique. Une déplétion supérieure à 90% des lymphocytes du sang périphérique est préférable.

A l'issue de ce traitement, une injection i.v. de lymphocytes T est réalisée aux patients à raison d'une dose préférentiellement comprise entre 10⁹ et 10¹¹ cellules. Les lymphocytes T ont dans ce cas été obtenus d'un donneur allogénique présentant le maximum de compatibilité des antigènes majeurs d'histocompatibilité. Cela peut être par exemple un germain HLA identique pour les situations dans lesquelles le risque génétique familial de retrouver les mêmes pathologies au sein de la famille n'est pas trop élevé. Les cellules réinjectées ont été préalablement transduites par un gène suicide comme le gène HSV1-TK et les cellules réinjectées contiennent au moins 90% de cellules transduites. Par ailleurs, l'ensemble des contrôles qualité de ces cellules sont réalisés (phénotype, analyse du répertoire).

Dans les quelques jours suivant l'injection, préférentiellement dans la semaine qui suit les injections, 48 heures après l'injection, un traitement gancyclovir à raison de 10 mg/kg deux fois par jour est administré au patient. Si des signes de GVH devaient survenir plus précocement, le traitement peut être envisagé de façon encore plus précoce. Le traitement dure au moins une semaine. A l'issue de ce traitement, le patient reste suivi, notamment pour vérifier la reconstitution des lymphocytes T.

Sont également suivis les signes indicateurs d'une réaction du greffon contre l'hôte comme par exemple les atteintes cutanées. Si de telles manifestations surviennent, un traitement curatif par le gancyclovir est entrepris.

Le traitement est répété et terminé lorsque la numération des lymphocytes a retrouvé des valeurs d'au moins 200 lymphocytes T par mm3.

En cas d'effets secondaires graves durant le traitement, comme par exemple une GVH qui ne pourrait être contrôlée par le gancyclovir, il est alors possible de retraiter le patient avec les médicaments permettant la destruction des lymphocytes T pour réinjecter ensuite ses propres cellules qui ont été cryopréservées au préalable. Par ailleurs, ces lymphocytes T ayant pour construction un marqueur membranaire, une injection de l'anticorps anti-marqueur aide à l'élimination des lymphocytes T, si nécessaire.

Pour d'autres types de pathologies autoimmunes, les lymphocytes qui sont réinjectés peuvent être des lymphocytes du patient transduits similairement par le gène HSV1-TK. Dans ces conditions, à l'issue de la réinjection des cellules, aucun traitement par le gancyclovir n'est institué. Après avoir constaté la reconstitution du pool de lymphocytes T du patient, lorsque surviendront des phases aiguës de poussées de la pathologie, un traitement gancyclovir sera institué de façon aussi précoce que possible après identification de ces poussées qui peuvent être par exemple des poussées d'arthrite inflammatoire pour le traitement d'une polyarthrite rhumatoïde ou d'atteinte neurologique et musculaire dans le cas de la sclérose en plaque.

### 3. Protocole thérapeutique de l'échange de lymphocytes T appliqué au traitement de la greffe d'organe.

Dans cette indication thérapeutique, le patient est conditionné comme précédemment décrit. Il est ensuite reconstitué par exemple avec ses propres cellules exprimant le gène suicide et en l'absence de traitement par le gancyclovir. Lorsque le patient a reconstitué son système immunitaire, la greffe d'organe proprement dite peut être entreprise. Dans les suites immédiates de la greffe, un traitement prophylactique d'environ une semaine par le gancyclovir peut être entrepris. Le patient est ensuite surveillé pour l'évolution des signes cliniques qui pourraient révéler un début de rejet du greffon. Dans ce cas, un nouveau traitement par le gancyclovir serait entrepris. Il est indiqué que les traitements immunosuppresseurs classiques comme par exemple la cyclosporine peuvent être utilisés dans cette situation.

Dans un autre mode thérapeutique, les lymphocytes peuvent provenir du donneur d'organe. Dans ce cas ils sont tolérants au greffon et le mode opératoire est donc un traitement GCV immédiatement après l'administration des lymphocytes T génétiquement modifiés dans le but de supprimer la GVH.

### 4. Protocole thérapeutique pour la prévention du vieillissement immunologique

Le système immunitaire est sujet au vieillissement, ce qui se caractérise par une mauvaise régulation de la réponse immune telle que l'apparition de maladies auto-immunes ou l'incapacité de monter une réponse immune efficace contre un nouvel antigène.

Dans cette application, le traitement est préventif. Quand le patient est encore jeune, des lymphocytes T lui sont prélevés. Il est facile et sans danger de prélever 10⁸ lymphocytes au cours d'une cytaphérèse.

Cette opération peut être répétée assez souvent à des intervalles de temps de l'ordre de quelques mois.

Il est actuellement admis que les sous-populations de lymphocytes T dites "mémoires" sont circulantes. Si tel n'était pas le cas, il faudrait les prélever chirurgicalement ou après mobilisation par un facteur de croissance ou autre.

Dans le mode de réalisation préférentiel, les lymphocytes T sont cultivés et amplifiés afin d'atteindre 10¹⁰ - 10¹¹ lymphocytes T.

Les cellules sont alors réparties et congelées pour plusieurs années. Quand le besoin s'en fait sentir : apparition d'un problème immunologique ou difficulté à montrer une réponse immune, les lymphocytes T sont décongelés et réinjectés au sujet.

Cette réinjection peut porter sur tout ou partie des lymphocytes T congelés et peut être précédée d'une ablation totale ou partielle des lymphocytes T circulants.

On peut tout à fait prévoir, par exemple de ne remplacer qu'une sous classe de lymphocytes T par une autre sous classe de lymphocytes T. Par exemple, il est tout à fait possible de ne détruire sélectivement que les lymphocytes CD8 (ou ceux porteurs d'un TCR particulier) et de trier les lymphocytes T préalablement congelés afin de n'injecter que la sous classe de lymphocytes T désirée, voire de dépléter sélectivement le greffon de telle ou telle sous classe de lymphocytes T.

On obtient alors le replacement des lymphocytes T du patient par des lymphocytes T plus jeunes et mieux à même d'assurer leurs fonctions. Dans le cas de maladies auto-immunes, les lymphocytes T alloréactifs sont détruits et remplacés par des lymphocytes T inoffensifs. Dans le cas d'une pathologie liée au vieillissement de la population des lymphocytes T, il s'agit d'une thérapeutique préventive. En effet, il s'agit réellement d'un "rajeunissement" des lymphocytes T et donc du système immunitaire.

### BIBLIOGRAPHIE

[1] P. Tiberghien, C.W. Reynolds, J. Keller, S. Spence, M. Deschaseaux, J.M. Certoux, E. Contassot, W.J. Murphy, R. Lyons, Y. Chiang, P. Hervé, D.L. Longo, F.W. Ruscetti, Ganciclovir treatment of herpes simplex thymidine kinase-transduced primary T lymphocytes: an approach for specific in vivo donor T-cell depletion after bone marrow transplantation?, Blood 84 (1994) 1333-1341.
[2] C. Bordignon, L.D. Notarangelo, N. Nobili, G. Ferrari, G. Casorati, P. Panina, E. Mazzolari, D. Maggioni, C. Rossi, P. Servida, a.l. et, Gene therapy in peripheral blood lymphocytes and bone marrow for ADA-immunodeficient patients, Science 270 (1995) 470-475.
[3] Anonymous, Human gene marker/therapy clinical protocols, Hum. Gene Ther. 8 (1997) 1499-1530.
[4] F. Moolten, Tumor sensitivity conferred by inserted herpes thymidine kinase genes: paradigm for prospective cancer control strategy., Cancer Res 46 (1986) 5276-5281.
[5] K. Culver, Z. Ram, S. Wallbridge, H. lshii, E. Oldfield, R. Blaese, *In vivo* gene transfer with retroviral vector-producer cells for treatment of experimental brain tumors., Science 256 (1992) 1550-1552.
[6] M. Caruso, Y. Panis, S. Gagandeep, D. Houssin, J. Salzmann, D. Klatzmann, Regression of established macroscopic liver metastases after *in situ* transduction of a suicide gene., Proc Natl Acad Sci USA 90 (1993) 7024-7028.
[7] C. Bonini, G. Ferrari, S. Verzeletti, P. Servida, E. Zappone, L. Ruggieri, M. Ponzoni, S. Rossini, F. Mavilio, C. Traversari, C. Bordignon, HSV-TK gene transfer into donor lymphocytes for control of allogeneic graft-versus-leukemia [see comments], Science 276 (1997) 1719-1724.
[8] J.L. Cohen, O. Boyer, B. Salomon, R. Onclercq, F. Charlotte, S. Bruel, G. Boisserie, D. Klatzmann, Prevention of graft-versus-host disease in mice using a suicide gene expressed in T lymphocytes, blood 89 (1997) 4636-4645.
[9] M. Helene, V. Lake-Bullock, J.S. Bryson, C.D. Jennings, A.M. Kaplan, Inhibition of graft-versus-host disease. Use of a T cell-controlled suicide gene, J Immunol 158 (1997) 5079-5082.
[10] F.L. Moolten, Drug sensitivity ("suicide") genes for selective cancer chemotherapy, Cancer Gene Ther 1 (1994) 279-287.
[11] J.A. Fyfe, P.M. Keller, P.A. Furman, R.L. Miller, G.B. Elion, Thymidine kinase from herpes simplex virus phosphorylates the new antiviral coumpound, 9-(2-hydroxyethoxymethyl)guanine., J Biol Chem 253 (1978) 8721-8727.
[12] Y. Nishiyama, F. Rapp, Anticellular effects of 9-(2-hydroxyethoxymethyl) guanine against herpes simplex virus-transformed cells, J. Gen. Virol. 45 (1979) 227-230.
[13] P.A. Furman, P.V. McGuirt, P.M. Keller, J.A. Fyfe, G.B. Elion, Inhibition by acyclovir of cell growth and DNA synthesis of cells biochemically transformed with herpesvirus genetic information, Virology 102 (1980) 420-430.
[14] R.L. Davidson, E.R. Kaufman, C.S. Crumpacker, L.E. Schnipper, Inhibition of herpes simplex virus transformed and nontransformed cells by acycloguanosine: mechanisms of uptake and toxicity, Virology 113 (1981) 9-19.
[15] G.B. Elion, The biochemistry and mechanism of action of acyclovir., J Antimicrob Chemother 12 Suppl B (1983) 9-17.
[16] A.K. Field, M.E. Davies, C. DeWitt, C. Perry, H.C. Liou, R. Germershausen, J. Karkas, W.T. Ashton, D.B. Jonhston, R.L. Tolman, 9-([2-hydroxy-1-(hydroxymethyl)ethoxy]methyl)guanine: a selective inhibitor of herpes group virus replication., Proc Natl Acad Sci USA 80 (1983) 4139-4143.
[17] S. Oliver, G. Bubley, C. Crumpacker, Inhibition of HSV-transformed murine cells by nucleoside analogs, 2'- NDG and 2'-nor-cGMP: mechanisms of inhibition and reversal by exogenous nucleosides, Virology 145 (1985) 84-93.
[18] M.H. St. Clair, C.U. Lambe, P.A. Furman, Inhibition by ganciclovir of cell growth and DNA synthesis of cells biochemically transformed with herpesvirus genetic information, Antimicrob Agents Chemother 31 (1987) 844-849.
[19] L.W. Bi, In vitro evidence that metabolic cooperation is responsible for the bystander effect observed with HSV tk retroviral gene therapy, Hum Gene Ther 4 (1993) 725-731.
[20] F.L. Moolten, J.M. Wells, R.A. Heyman, R.M. Evans, Lymphoma regression induced by ganciclovir in mice bearing a herpes thymidine kinase transgene, Hum Gene Ther 1 (1990) 125-134.
[21] Z. Ezzeddine, R. Martuza, D. Platika, M. Short, M. Malick, A. Choi, X. Breakefield, Selective killing of glioma cells in culture and *in vivo* by retrovirus transfer of the herpes simplex virus thymidine kinase gene., New Biol 3 (1991) 608-614.
[22] D. Barba, J. Hardin, R. Jasodhara, F. Gagae, Thymidine kinase-mediated killing of rat brain tumors., J Neurosurg 79 (1993) 729-735.
[23] S. Chen, H. Shine, J. Goodman, R. Grossman, S. Woo, Gene therapy for brain tumors: regression of experimental gliomas by adenovirus-mediated gene transfer *in vivo.,* Proc Natl Acad Sci USA 91 (1994) 3054-3057.
[24] S.M. Freeman, C.N. Abboud, K.A. Whartenby, C.H. Packman, D.S. Koeplin, F.L. Moolten, G.N. Abraham, The "bystander effect": tumor regression when a fraction of the tumor mass is genetically modified, Cancer Res 53 (1993) 5274-5283.
[25] Z. Ram, K.W. Culver, E.M. Oshiro, J.J. Viola, H.L. DeVroom, E. Otto, Z. Long, Y. Chiang, G.J. McGarrity, L.M. Muul, D. Katz, R.M. Blaese, E.H. Oldfield, Therapy of malignant brain tumors by intratumoral implantation of retroviral vector-producing cells [see comments], Nat Med 3 (1997) 1354-1361.
[26] P. Salmon, O. Boyer, P. Lores, J. Jami, D. Klatzmann, Characterization of an intronless CD4 minigene expressed in mature CD4 and CD8 T cells, but not expressed in immature thymocytes, J. Immunol. 156 (1996) 1873-1879.
[27] C. Bordignon, C. Bonini, S. Verzeletti, N. Nobili, D. Maggioni, C. Traversari, R. Giavazzi, P. Servida, E. Zappone, E. Benazzi, M. Bernardi, F. Porta, G. Ferrari, F. Mavilio, S. Rossini, R.M. Blaese, F. Candotti, Transfer of the HSV-tk gene into donor peripheral blood lymphocytes for in vivo modulation of donor anti-tumor immunity after allogeneic bone marrow transplantation, Hum Gene Ther 6 (1995) 813-819.
[28] P. Tiberghien, J.Y. Cahn, A. Brion, E. Deconinck, E. Racadot, P. Herve, N. Milpied, B. Lioure, E. Gluckman, P. Bordigoni, W. Jacob, Y. Chiang, S. Marcus, C. Reynolds, D. Longo, Use of donor T-lymphocytes expressing herpes-simplex thymidine kinase in allogeneic bone marrow transplantation: a phase I-II study, Hum Gene Ther 8 (1997) 615-624.

## Revendications

1. Utilisation d'une population de lymphocytes T, pour la préparation d'une composition destinée au traitement d'une immunopathologie médiée par les lymphocytes T chez un sujet ayant subi une déplétion spécifique de ses lymphocytes T ou de sous-populations de lymphocytes T, sans déplétion de ses autres cellules hématopoïétiques, dont les cellules souches.

2. Utilisation selon la revendication 1 **caractérisée en ce que** la population de lymphocytes T comprend des lymphocytes T autologues ou syngéniques vis-à-vis du sujet.

3. Utilisation selon la revendication 1 **caractérisée en ce que** la population de lymphocytes T comprend des lymphocytes T allogéniques vis-à-vis du sujet.

4. Utilisation selon l'une des revendications 1 à 3 **caractérisée en ce que** la population de lymphocytes T comprend des lymphocytes T modifiés génétiquement.

5. Utilisation selon la revendication 4 **caractérisée en ce que** les lymphocytes comprennent un acide nucléique codant pour un produit toxique.

6. Utilisation selon la revendication 5 **caractérisée en ce que** l'acide nucléique code pour une protéine ayant une toxicité conditionnelle, telle que par exemple la thymidine kinase.

7. Utilisation selon les revendications 4 ou 5 **caractérisée en ce que** les lymphocytes comprennent un acide nucléique codant pour un produit thérapeutique.

8. Utilisation selon l'une des revendications 4 à 7 **caractérisée en ce que** les lymphocytes sont modifiés génétiquement au moyen d'un vecteur viral.

9. Utilisation selon la revendication 8 **caractérisée en ce que** le vecteur viral est un vecteur rétroviral ou AAV, de préférence rétroviral.

10. Utilisation selon l'une des revendications 1 à 4 **caractérisée en ce que** la population de lymphocytes T comprend des lymphocytes T modifiés immunologiquement.

11. Utilisation selon la revendication 10 **caractérisée en ce que** la modification immunologique comprend la modification du répertoire des lymphocytes T de la composition.

12. Utilisation selon l'une des revendications 1, 3 à 4 ou 10 **caractérisée en ce que** la population de lymphocytes T comprend des lymphocytes T procurant une immunité accrue.

13. Utilisation selon la revendication 1 **caractérisée en ce que** la déplétion des lymphocytes T est réalisée par traitement du sujet en présence d'un ou plusieurs agents immunosuppresseurs.

14. Utilisation selon la revendication 13 **caractérisée en ce que** la déplétion des lymphocytes T est réalisée par traitement du sujet en présence d'un agent immunossupresseur spécifique des lymphocytes T.

15. Utilisation selon la revendication 13 **caractérisée en ce que** l'agent immunossupresseur est un sérum ou un anticorps anti-CD3, CD4 et/ou CD8.

16. Utilisation selon la revendication 6 comprenant en outre l'administration au sujet d'une drogue susceptible d'être transformée par la protéine en métabolite toxique.

17. Produit comprenant:
- un ou plusieurs agents immunosuppresseurs, et
- une composition comprenant une population de lymphocytes T modifiée immunologiquement par la suppression *in vitro* ou *ex vivo* de clones de lymphocytes impliqués dans des réponses immunopathologiques et/ou de sous-populations de lymphocytes T ayant des propriétés immunopathologiques particulières,
en vue d'une utilisation séparée ou espacée dans le temps.

18. Produit selon la revendication 17, **caractérisé en ce que**
- la composition comprend une population de lymphocytes T comprenant un gène suicide, et **en ce qu'**il comprend en outre
- une drogue susceptible d'être transformée par le gène suicide en métabolite toxique.

19. Procédé de préparation d'une composition de lymphocytes T modifiés comprenant:
- le prélèvement de lymphocytes T chez un sujet, et
- la modification du répertoire de ces lymphocytes par suppression *in vitro* ou *ex vivo* de clones de lymphocytes impliqués dans des réponses immunopathologiques et/ou de sous-populations de lymphocytes T ayant des propriétés immunopathologiques particulières.

20. Procédé selon la revendication 19 **caractérisé en ce que** le trou du répertoire est réalisé par déplétion des lymphocytes T spécifiques d'antigènes impliqués dans des pathologies.

21. Utilisation selon la revendication 1, pour la préparation d'une composition destinée à la prévention ou au traitement des immunopathologies, notamment dans le cadre de greffe d'organe ou de cellules.

22. Utilisation selon la revendication 1, pour la préparation d'une composition destinée à la prévention ou au traitement du vieillissement du système immunitaire cellulaire.

## Patentansprüche

1. Verwendung einer Population von T-Lymphocyten zur Herstellung einer Zusammensetzung, die zur Behandlung einer von den T-Lymphocyten vermittelten Immunkrankheit bei einer Person bestimmt ist, die einer spezifischen Depletion ihrer T-Lymphocyten oder Subpopulationen von T-Lymphocyten ohne Depletion ihrer anderen hämatopoietischen Zellen, darunter die Stammzellen, unterzogen wird.

2. Verwendung gemäß Anspruch 1, **dadurch gekennzeichnet, dass** die Population von T-Lymphocyten bezogen auf die Person autologe oder syngene T-Lymphocyten umfasst.

3. Verwendung gemäß Anspruch 1, **dadurch gekennzeichnet, dass** die Population von T-Lymphocyten bezogen auf die Person allogene T-Lymphocyten umfasst.

4. Verwendung gemäß einem der Ansprüche 1 bis 3, **dadurch gekennzeichnet, dass** die Population von T-Lymphocyten genetisch modifizierte T-Lymphocyten umfasst.

5. Verwendung gemäß Anspruch 4, **dadurch gekennzeichnet, dass** die T-Lymphocyten eine für ein toxisches Produkt codierende Nucleinsäure umfassen.

6. Verwendung gemäß Anspruch 5, **dadurch gekennzeichnet, dass** die Nucleinsäure für ein Protein codiert, das eine bedingte Toxizität besitzt, wie zum Beispiel die Thymidin-Kinase.

7. Verwendung gemäß Ansprüchen 4 oder 5, **dadurch gekennzeichnet, dass** die Lymphocyten eine für ein therapeutisches Produkt codierende Nucleinsäure umfassen.

8. Verwendung gemäß einem der Ansprüche 4 bis 7, **dadurch gekennzeichnet, dass** die Lymphocyten mit Hilfe eines viralen Vektors genetisch modifiziert sind.

9. Verwendung gemäß Anspruch 8, **dadurch gekennzeichnet, dass** der virale Vektor ein retroviraler oder AAV vorzugsweise ein retroviraler Vektor ist.

10. Verwendung gemäß einem der Ansprüche 1 bis 4, **dadurch gekennzeichnet, dass** die Population von T-Lymphocyten immunologisch modifizierte T-Lymphocyten umfasst.

11. Verwendung gemäß Anspruch 10, **dadurch gekennzeichnet, dass** die immunologische Modifikation die Modifikation des Repertoires der T-Lymphocyten der Zusammensetzung umfasst.

12. Verwendung gemäß einem der Ansprüche 1, 3 bis 4 oder 10, **dadurch gekennzeichnet, dass** die Population von T-Lymphocyten eine erhöhte Immunität bereitstellende T-Lymphocyten umfasst.

13. Verwendung gemäß Anspruch 1, **dadurch gekennzeichnet, dass** die Depletion der T-Lymphocyten durch Behandlung der Person in Gegenwart eines oder mehrerer Immunsuppressiva durchgeführt ist.

14. Verwendung gemäß Anspruch 13, **dadurch gekennzeichnet, dass** die Depletion der T-Lymphocyten durch Behandlung der Person in Gegenwart eines für T-Lymphocyten spezifischen Immunsuppressivums durchgeführt ist.

15. Verwendung gemäß Anspruch 13, **dadurch gekennzeichnet, dass** das Immunsuppressivum ein anti-CD3, CD4 und/oder CD8 Serum oder Antikörper ist.

16. , Verwendung gemäß Anspruch 6, außerdem umfassend die Verabreichung eines Medikaments an die Person, das geeignet ist, von dem Protein in einen toxischen Metaboliten umgewandelt zu werden.

17. Produkt, umfassend:
- ein oder mehrere Immunsuppressiva, und
- eine Zusammensetzung, umfassend eine Population von T-Lymphocyten, die durch die *in vitro* oder *ex vivo* Suppression von Lymphocyten-Klonen, die an immunpathologischen Antworten beteiligt sind, und/oder von Subpopulationen von T-Lymphocyten mit besonderen immunpathologischen Eigenschaften, immunologisch modifiziert sind,
im Hinblick auf eine separate oder in zeitlichen Abständen erfolgende Verwendung.

18. Produkt gemäß Anspruch 17, **dadurch gekennzeichnet, dass**
- die Zusammensetzung eine Population von T-Lymphocyten umfasst, die ein Selbstmordgen umfassen, und dass das Produkt außerdem umfasst
- ein Medikament, das geeignet ist, von dem Selbstmordgen in einen toxischen Metaboliten umgewandelt zu werden.

19. Verfahren zur Herstellung einer Zusammensetzung von modifizierten T-Lymphocyten, umfassend:
- die Entnahme von T-Lymphocyten bei einer Person, und
- die Modifikation des Repertoires dieser Lymphocyten durch *in vivo* oder *ex vivo* Suppression von Lymphocyten-Klonen, die an immunpathologischen Antworten beteiligt sind, und/oder von Subpopulationen von T-Lymphocyten mit besonderen immunpathologischen Eigenschaften.

20. Verfahren gemäß Anspruch 19, **dadurch gekennzeichnet, dass** die Lücke im Repertoire durch Depletion der T-Lymphocyten, die für bei Krankheiten beteiligte Antigene spezifisch sind, geschaffen ist.

21. Verwendung gemäß Anspruch 1 zur Herstellung einer Zusammensetzung, die zur Prävention oder zur Behandlung von Immunkrankheiten, besonders im Rahmen von Organ- oder Zelltransplantationen, bestimmt ist.

22. Verwendung gemäß Anspruch 1 zur Herstellung einer Zusammensetzung, die zur Prävention oder zur Behandlung der Alterung des zellulären Immunsystems bestimmt ist.

## Claims

1. The use of a population of T lymphocytes for the preparation of a composition for treating an immunopathology mediated by T lymphocytes in a subject whose T lymphocytes or sub-populations of T lymphocytes have been specifically depleted, without depleting his other hematopoietic cells, including stem cells.

2. The use of claim 1, wherein the population of T lymphocytes comprises T lymphocytes which are autologous or syngeneic for the subject.

3. The use of claim 1, wherein the population of T lymphocytes comprises T lymphocytes which are allogeneic for the subject.

4. The use of any one of claims 1 to 3, wherein the population of T lymphocytes comprises genetically modified T lymphocytes.

5. The use of claim 4, wherein the lymphocytes comprise a nucleic acid encoding a toxic product.

6. The use of claim 5, wherein the nucleic acid encodes a protein with conditional toxicity, such as for instance thymidine kinase.

7. The use of claim 4 or 5, wherein the lymphocytes comprise a nucleic acid encoding a therapeutic product.

8. The use of any one of claims 4 to 7, wherein the lymphocytes are genetically modified with a viral vector.

9. The use of claim 8, wherein the viral vector is a retroviral or AAV vector, preferably a retroviral vector.

10. The use of any one of claims 1 to 4, wherein the population of T lymphocytes comprises immunologically modified T lymphocytes.

11. The use of claim 10, wherein the immunological modification comprises the modification of the repertoire of T lymphocytes in the composition.

12. The use of any one of claims 1, 3 to 4 or 10, wherein the population of T lymphocytes comprises T lymphocytes providing an increased immunity.

13. The use of claim 1, wherein the depletion ofT lymphocytes is performed by treating the subject in the presence of one or several immunosuppressive agents.

14. The use of claim 13, wherein the depletion of T lymphocytes is performed by treating the subject in the presence of a T lymphocyte-specific immunosuppressive agent.

15. The use of claim 13, wherein the immunosuppressive agent is an anti-CD3, CD4 and/or CD8 serum or antibody.

16. The use of claim 6, further comprising administering to the subject a drug that can be transformed by the protein into a toxic metabolite.

17. A product comprising:
- one or several immunosuppressive agents, and
- a composition comprising a population of T lymphocytes immunologically modified by in vitro or ex vivo suppression of clones of lymphocytes involved in immunopathological responses and/or of sub-populations of T lymphocytes having particular immunopathological properties,
for separate or sequential use.

18. The product of claim 17, wherein:
- the composition comprises a population of T lymphocytes comprising a suicide gene, and wherein the product further comprises
- a drug that can be transformed by the suicide gene into a toxic metabolite.

19. A method for preparing a composition of modified T lymphocytes, comprising:
- collecting T lymphocytes from a subject, and
- modifying the repertoire of said lymphocytes by in vitro or ex vivo suppression of clones of lymphocytes involved in immunopathological responses and/or of sub-populations of T lymphocytes having particular immunopathological properties.

20. The method of claim 19, wherein the hole in the repertoire in performed by depleting T lymphocytes specific for antigens involved in pathologies.

21. The use of claim 1, for the preparation of a composition for preventing or treating immunopathologies, in particular during organ or cell transplantation.

22. The use of claim 1, for the preparation of a composition for preventing or treating aging of the cellular immune system.
